# EUROPEAN PATENT APPLICATION

(11) **EP 4 273 174 A1**
(43) Date of publication of application: **08.11.2023**
(21) Application number: 21913269.3
(22) Date of filing: 26.09.2021
(51) Int. Cl.: C07K 19/00, C12N 15/62, C12N 5/10, A61P 35/00, A61K 39/12

(54) **HUMAN PAPILLOMAVIRUS TYPE 31 CHIMERIC PROTEIN AND USE THEREOF**

(30) Priority: 04.01.2021 CN 202110002620
(71) Applicant: Institute of Basic Medical Sciences, Chinese Academy of Medical Sciences, Beijing 100005 (CN)
(72) Inventor: XU, Xuemei, Beijing 100005 (CN); HAO, Yaru, Beijing 100005 (CN); ZHANG, Ting, Beijing 100005 (CN); MA, Mingrao, Beijing 100005 (CN)
(74) Representative: EP&C
(86) International application number: PCT/CN2021/120603
(87) International publication number: WO 2022/142524

(57) **Abstract**

The present invention relates to a human papillomavirus type 31 chimeric protein and a use thereof. Specifically, the present invention relates to a human papillomavirus chimeric protein, containing or being composed of an HPV31 L1 protein or HPV31 L1 protein mutant, and a polypeptide derived from an HPV73 L2 protein and inserted into the HPV31 L1 protein or HPV31 L1 protein mutant, wherein the HPV31 L1 protein is as shown in SEQ ID No. 1, and the HPV73 L2 protein is as shown in SEQ ID No. 2.

## Description

### FIELD OF THE INVENTION

The present invention relates to the field of biotechnology. Specifically, the present invention relates to a human papillomavirus chimeric protein, and a pentamer or a virus-like particle formed thereby, as well as use of the human papillomavirus chimeric protein, the pentamer or the virus-like particle thereof in the preparation of a vaccine for the prevention of papillomavirus infection and infection-induced diseases in a subject.

### BACKGROUND OF THE INVENTION

Human papillomaviruses (HPVs) are a class of envelope-free small DNA viruses that infect epithelial tissues. At present, more than 200 types of HPVs have been identified, among which more than 40 types mainly infect the perianal, urogenital and oropharyngeal mucous membrane and adjacent skin. According to the nature of infection-induced lesions, they are classified into carcinogenic types that induce malignant tumors (HPV16/-18/-31/-33/-45/-52/-58, etc.) and low-risk types that induce verrucous hyperplasia (HPV6/-11, etc.). Molecular epidemiological studies have found that persistent infection with carcinogenic HPVs can induce about 100% of cervical cancer, 88% of anal cancer, 70% of vaginal cancer, 50% of penile cancer, 43% of vulva cancer, and 72% of head and neck cancer. At present, more than 20 types of HPVs have been identified, and the 12 types commonly found in cervical cancer tissues, such as HPV16/-18/-31/-33/-35/-39/-45/-51/-52/-56/-58/-59, are also known as high-risk HPVs (HR-HPVs). HR-HPVs induce an accumulative total of 95.2%~96.5% of cervical cancers; other carcinogenic HPVs are relatively rare, with a detection rate of less than 0.5% for a single type except for HPV68. HPV16 and HPV18 have the highest detection rates in cervical cancer worldwide, which are 55.4% and 14.6%, respectively. HPV31 is a relatively common HR-HPV worldwide. Its detection rate in cervical cancer tissues is 3.5%, ranking sixth; its detection rate in high grade precancerous cervical lesions is 10.4%, ranking third, just next to HPV16 (45.1%) and HPV52 (11%); its detection rate in cytologically normal cervical tissues is 1.3%, ranking third, just next to HPV16 (2.9%) and HPV58 (1.5%). It is worth noting that in some developed areas, the detection rate of HPV31 in high grade precancerous cervical lesions is as high as 12.4%, just next to HPV16 (46.8%); in addition, in Latin America, the detection rate of HPV31 in cytologically normal cervical tissues is 1.2%, just next to HPV16 (3.3%).

The C-terminus of HPV L1 protein contains a nuclear localization signal, and after the L1 protein is expressed in eukaryotic cells, it is introduced into the nucleus through the mediation of the nuclear localization signal, and assembled into virus-like particles in the nucleus. Researchers have found that the L1 protein could be distributed in the cytoplasm after translation by removing the nuclear localization signal by deletion. Therefore, on the basis of not affecting the C-terminus helix 5 domain of L1 protein involved in VLP assembly, the C-terminus truncated L1 gene obtained by removing the nuclear localization signal was mainly distributed in the cytoplasm after expression in eukaryotic expression systems (such as insect cells), which was advantageous to cell disruption and downstream purification. Studies with insect cell expression systems have found that the mutant of bovine papillomavirus type 1 (BPV1) L1 with a 24-amino acid truncation at the C-terminus, the mutant of HPV16 L1 with a 23-amino acid truncation the C-terminus, and the mutant of HPV58 L1 with a 25-amino acid truncation the C-terminus, did not affect the activity of L1 protein assembly into VLP, and the assembly efficiency of the BPV1 L1 truncated protein mutant increased by 3 folds, and the expression of HPV58 L1 truncated protein increased by 2 folds. The effects of truncated mutants of other types on expression level, assembly activity and yield have not been reported. All the L1 genes for the expression of L1 VLP by *E. coli* expression system reported so far have C-terminus with natural complete sequence.

According to the difference in the amino acid sequence of L1, there are many different variants of L1 of each HPV type. Reported data show that there are differences in the expression levels of HPV16L1 variants in insect cells and yeast cells. A report analyzed the expression levels of L1 VLP of five strains of 16L1 variants in insect cells, and found that the expression level of L1 and VLP yield of two of these variants (Phil1 and Fra63) were significantly higher than those of the original L1 control group (in Phil1 and Fra63, the expression levels increased by 32 folds and 16 folds compared to the original, and the yields increased by 39 folds and 42 folds, respectively). In another variant (Alg1), the expression level increased by 8 folds compared to the original, and the yield increased by 24 folds. The expression levels and yields in the other two variants were comparable to those of the original. The expression levels of VLP in two 16L1 variants (B27 and T3) were analyzed by a yeast expression system, and it was found that the VLP yields of both two variants were significantly higher than that of the original control group. Amino acid analysis found that the primary amino acid sequences of the above seven strains of 16L1 variants were all different. The two variants with relatively high expression levels in yeast and the three variants with relatively high expression levels in insect cells had common characteristics of having the same the amino acids in positions 202 and 266 (Asp and Ala, respectively), however, the expression level and VLP yield of Fra25 with the same characteristics were very low (comparable to those of the control group), indicating that the characteristic constituent amino acids in different variants may affect the expression level of L1, which is unpredictable. The above data show that the sequences of variants with increased expression amount of VLP can be found by analysis of the expression levels of different variants, and used for the production of L1 VLP vaccines, which is expected to reduce the production cost of vaccines. The effects of variant sequences of other types on expression levels have not been reported.

Research using *E. coli* expression system to study the expression of HPV 16L1 with complete C-terminus showed that truncation of 4, 6, 8, 9 and 10 amino acids, respectively, at the N-terminus did not affect the assembly of L1 VLP (X. Chen et al, Journal of Molecular Biology, 2001). The expression levels of mutants with N-terminus truncation of a total of 9 types of L1 and the expression levels of L1 with full-length N-terminus of the corresponding types were compared and analyzed using an *E. coli* expression system, among which, both of the two mutants with N-terminus truncation of 16L1 (△N5, ΔN10) showed significantly increased expression levels compared with the control group; one of the two mutants with N-terminus truncation of 18L1 (△N5, △N10) showed significantly increased expression level (△NS) while the other showed significantly decreased expression level (△N10); both of the two mutants with N-terminus truncation of 31L1 (△N5 and ΔN10) showed expression levels comparable to the control group; one of the three mutants with N-terminus truncation of 33L1 (△N5, △N10, △N15) showed significantly increased expression level (△N10), while the other two showed decreased expression levels; both of the two mutants with N-terminus truncation of 45L1 (△N4 and △N9) showed reduced expression levels; one of the four mutants with N-terminus truncation of 58L1 (△N5, △N10, △N15, △N19) showed significantly increased expression level (△N15), while the other three showed expression level comparable to the control group; all of the three mutants with N-terminus truncation of 52L1 (△N5, △N10, △N15) showed significantly increased expression levels; one of the three mutants with N-terminus truncation of 6L1 (△N3, △N6, △N9) showed significantly increased expression level (△N6), one showed unchanged expression level (△N9), and one showed decreased expression level (△N3); one of the two mutants with N-terminus truncation of 11 1 (△N5, △N9) showed significantly increased expression level (△N5), and the other showed unchanged expression level (△N9) (M. Wei et al, Emerging Microbes & Infections, 2018). The above data show that the mutants with truncation at the N-terminus of L1 can affect the expression level of the protein in *E. coli,* and the effect of the length of N-terminus truncation on the expression level is irregular and unpredictable. No studies have been performed on the expression levels of mutants with N-terminus truncation in insect cells. In addition, no studies have been found to study the effect of the strategy of N-terminus truncation on the expression level of truncated protein in eukaryotic expression systems by performing N-terminus truncation on the mutants of L1 with C-terminus truncation.

L1 VLP is an icosahedron with a diameter of 55 nm, assembled from 72 L1 pentamers (360 L1 monomers). L1-dependent neutralizing antibody epitopes are regularly and densely arranged on its surface at a certain interval, and each epitope is repeated for 360 times, so L1VLP is advantageous to the crosslinking of BCR and to the production of high-titer neutralizing antibodies. Animal experiments and clinical research data show that HPV L1VLP can induce the persistent production of high-titer L1-specific neutralizing antibodies. After the application of three marketed VLP vaccines in more than 100 countries and regions worldwide, no escape from any variant within a type is observed, indicating that VLP induces responses against multiple L1 epitopes.

In addition, VLP can also be used as a carrier for epitope peptide vaccines. The HPV16 cVLP vaccines with surface display of 16RG-1, 58RG-1, 33RG-1 and 31RG-1 constructed using HPV16 L1 as the carrier was relatively successful, and could simultaneously induce different types of RG-1-dependent cross-neutralizing antibodies and HPV16L1-dependent type-specific neutralizing antibodies, in which the titer of HPV16 neutralizing antibodies was comparable to that of 16L1VLP, the titer of each type of RG-1-dependent cross-neutralizing antibodies was relatively high, and the neutralization range covered relatively more types (more than 10 types). Some research data of HPV16 cVLP vaccines were also reported in other literatures, and the chimeric epitopes included 16RG-1 and other epitope regions of 16L2. However, due to the difference in the insertion site, insertion strategy and length of the selected epitope, the obtained cross-neutralization activity induced by 16cVLP was relatively poor. The 16VLP in Cervarix (HPV16/18 L1VLP) was replaced with 16cVLP to prepare a 16cVLP/18VLP combination vaccine, and preliminary studies showed that the vaccine could not only induce high titers of HPV16/18 neutralizing antibodies, but also induce cross-protective activity against HPV58. This suggests that the development of a new generation of prophylactic vaccines using HPV cVLP vaccines is expected to expand the range of vaccine protection and reduce the cost of vaccines at the same time. Based on the success of its bivalent vaccine Cervarix, GSK conducted research on HPV16 cVLP and HPV18 cVLP vaccines, but the data showed that the expression amount of its 16cVLP embedded with 33RG-1 was relatively low, and the cross-neutralization range was not ideal. The cross-neutralization range of its 18cVLP vaccine embedded with 33RG-1 only covered 7 types (4 high-risk types, 2 low-risk types, and 1 skin type), and except for the relatively high neutralization titer against HPV58, the titers of cross-neutralizing antibodies against the other 6 types were relatively low, but its immune activity was significantly better than that of 18cVLP embedded with 45RG-1 reported by Huber et al. (B. Huber et al., PLOS ONE, 2017; M. Boxus et al., Journal of Virology, 2016). The above data suggest that 16cVLP has been relatively successful, while the experimental research of 18cVLP is in a preliminary stage, and it is still necessary to further optimize the inserted epitopes and insertion sites in order to further improve the range covered by its immunoprotective activity; other types of cVLP vaccines have not been reported.

According to the research data of HPV16/18 cVLP vaccines reported so far, it can be seen that the research of cVLP vaccines faces many challenges. Firstly, it is necessary to select chimeric conserved L2 epitopes with strong immunogenicity, including RG-1 epitopes and other conserved epitopes in the L2 protein. At present, the selection of RG-1 epitopes is empirical, and RG-1 epitopes of dominantly epidemic strain types are often selected instead of RG-1 epitopes with strong immunogenicity, mainly due to the lack of data on the comparison of immunogenicity between different types of RG-1 epitopes. Secondly, the length of the RG-1 epitope peptide, i.e., the sequences flanking the epitope core sequence, has an influence on the correct display of the epitope on the surface of the chimeric protein. Thirdly, the differences in insertion site and insertion mode of epitope peptides have a great influence on the assembly and activity of chimeric proteins. The differences in insertion site include insertion sites in different surface areas of L1, and insertion sites at different positions in the same surface area. The differences in insertion mode include direct insertion, substitution insertion, and whether the backbone amino acids in the insertion site region are modified (including whether linkers are added). Fourthly, in addition to the above three challenges, the influence of HPV31 L1 VLP carrier on the tolerance and immune activity of the chimeric epitope also poses many other challenges, mainly because the structural characteristics of HPV31 L1 VLP and the main neutralizing antibody epitope regions are not clear. The insertion sites of the current successful HPV16 and 18 cVLPs are all in DE loop. Given that the suitable sites on HPV31 L1 VLP for the display of exogenous epitopes are not clear, if the insertion site is not properly selected, the immunogenicity of HPV31 L1VLP backbone will be affected, and the titer of 31cVLP-induced HPV31 neutralizing antibodies will be significantly lower than that of HPV31 L1 VLP. Even if chimeric epitope-dependent broad-spectrum neutralizing antibodies are obtained, 31cVLP still loses its immunoprotective advantage against HPV31 in the preparation of mixed vaccines of different types of cVLPs.

Therefore, at present, it is necessary to develop a new type of HPV cVLP, which is firstly required to have high expression amount and advantages in research and development, and also required to simultaneously induce high titers of neutralizing antibodies against the carrier type, meanwhile inducing cross-neutralizing antibodies with relatively strong activities, among which it is better that the titer of type-specific L1-dependent neutralizing antibodies is comparable to that induced by the L1 VLP of its corresponding type, so as to maintain the protective advantage for the backbone type in the study of mixed vaccines of different types of cVLPs; the cross-neutralization activity should cover many types with high titers, and its dominant cross-neutralization type should be distinctive from other types of cVLPs reported so far.

### SUMMARY OF THE INVENTION

In view of this, the object of the present invention is to provide a papillomavirus chimeric protein for the preparation of a vaccine for the prevention of papillomavirus infection and infection-induced diseases in a subject.

The inventors have unexpectedly found that appropriate truncation, point mutation and/or amino acid modification at the C-terminus of HPV31 L1 protein backbone can increase its expression level to varying degrees without affecting its activity of assembling into VLP. The insertion of HPV type 73 L2 protein polypeptide into the surface region of the full-length or truncated HPV type 31 L1 protein can improve the immunogenicity of HPV type 73 L2 protein polypeptide. The obtained chimeric protein can be expressed at a high level in an *E. coli* or insect cell expression system. The chimeric protein can be assembled into VLP, and can induce a broad-spectrum protective immune response against multiple types of HPVs from different genera/subgenera.

Therefore, in a first aspect, the present invention provides a human papillomavirus chimeric protein comprising or consisting of a HPV type 31 L1 protein or a mutant of the HPV type 31 L1 protein, and a polypeptide from an HPV type 73 L2 protein inserted into the HPV type 31 L1 protein or the mutant of the HPV type 31 L1 protein, wherein the HPV type 31 L1 protein is as shown in SEQ ID No. 1, and the HPV type 73 L2 protein is as shown in SEQ ID No. 2.

In a preferred embodiment of the human papillomavirus chimeric protein according to the present invention, the HPV type 31 L1 protein is from, for example but not limited to, the L1 proteins P17388.1, AEI61021.1, AEI60949.1, AAA92894.1, AIG59245.1, AIG59235.1, etc., from the original HPV31 or variant strains in the NCBI database. Preferably, the amino acid sequence of the HPV type 31 L1 protein is as shown in SEQ ID No. 1.

In a further preferred embodiment of the human papillomavirus chimeric protein according to the present invention, compared with the HPV type 31 L1 protein as shown in SEQ ID No. 1, the mutant of the HPV type 31 L1 protein according to the present invention comprises:
one or more substitution mutations selected from the group consisting of T274N, R475G, R483G, R496G, K477S, K497S, K501S, K479A, K482A, K498A, K495G, K500G and R473G; and/or
truncation mutation of 2, 4, 5, 8 or 10 amino acids truncated at the N-terminus; and/or
truncation mutation of 29 amino acids truncated at the C-terminus.

In the representation of the substitution mutation used herein, the number in the middle represents the amino acid position compared to the control sequence (e.g., the amino acid sequence as shown in SEQ ID No. 1), the letter preceding the number represents the amino acid residue before mutation, and the letter succeeding the number represents the amino acid residue after mutation.

In a further preferred embodiment, the mutant of the HPV type 31 L1 protein is selected from the group consisting of:
a mutant with a substitution of threonine (T) at position 274 of the amino acid sequence as shown in SEQ ID No. 1 to asparagine (N), and the sequence of the mutant is as shown in SEQ ID No. 3;
a mutant with a substitution of threonine (T) at position 274 of the amino acid sequence as shown in SEQ ID No. 1 to asparagine (N) and a 4-amino acid truncation at the N-terminus of the amino acid sequence, and the sequence of the mutant is as shown in SEQ ID No. 4;
a mutant with a 29-amino acid truncation at the C-terminus of the amino acid sequence as shown in SEQ ID No. 1, and the sequence of the mutant is as shown in SEQ ID No. 5;
a mutant with a substitution of threonine (T) at position 274 of the amino acid sequence as shown in SEQ ID No. 1 to asparagine (N) and a 29-amino acid truncation at the C-terminus of the amino acid sequence, and the sequence of the mutant is as shown in SEQ ID No. 6;
a mutant with a substitution of threonine (T) at position 274 of the amino acid sequence as shown in SEQ ID No. 1 to asparagine (N), a 29-amino acid truncation at the C-terminus of the amino acid sequence, and a 2-amino acid truncation at the N-terminus of the amino acid sequence, and the sequence of the mutant is as shown in SEQ ID No. 7;
a mutant with a substitution of threonine (T) at position 274 of the amino acid sequence as shown in SEQ ID No. 1 to asparagine (N), a 29-amino acid truncation at the C-terminus of the amino acid sequence, and a 4-amino acid truncation at the N-terminus of the amino acid sequence, and the sequence of the mutant is as shown in SEQ ID No. 8;
a mutant with a substitution of threonine (T) at position 274 of the amino acid sequence as shown in SEQ ID No. 1 to asparagine (N), a 29-amino acid truncation at the C-terminus of the amino acid sequence, and a 5-amino acid truncation at the N-terminus of the amino acid sequence, and the sequence of the mutant is as shown in SEQ ID No. 9;
a mutant with a substitution of threonine (T) at position 274 of the amino acid sequence as shown in SEQ ID No. 1 to asparagine (N), a 29-amino acid truncation at the C-terminus of the amino acid sequence, and a 8-amino acid truncation at the N-terminus of the amino acid sequence, and the sequence of the mutant is as shown in SEQ ID No. 10;
a mutant with a substitution of threonine (T) at position 274 of the amino acid sequence as shown in SEQ ID No. 1 to asparagine (N), a 29-amino acid truncation at the C-terminus of the amino acid sequence, and a 10-amino acid truncation at the N-terminus of the amino acid sequence, and the sequence of the mutant is as shown in SEQ ID No. 11;
a mutant with a substitution of threonine (T) at position 274 of the amino acid sequence as shown in SEQ ID No. 1 to asparagine (N), a 4-amino acid truncation at the N-terminus of the amino acid sequence, and substitutions of arginine (R) at positions 475, 483 and 496 of the amino acid sequence to glycine (G), lysine (K) at positions 477, 497 and 501 to serine (S), lysine (K) at positions 479, 482 and 498 to alanine (A), and lysine (K) at positions 495 and 500 to glycine (G), and the sequence of the mutant is as shown in SEQ ID No. 12;
a mutant with a substitution of threonine (T) at position 274 of the amino acid sequence as shown in SEQ ID No. 1 to asparagine (N), a 4-amino acid truncation at the N-terminus of the amino acid sequence, and substitutions of arginine (R) at positions 473, 475, 483 and 496 of the amino acid sequence to glycine (G), lysine (K) at positions 477, 497 and 501 to serine (S), lysine (K) at positions 479, 482 and 498 to alanine (A), and lysine (K) at positions 495 and 500 to glycine (G), and the sequence of the mutant is as shown in SEQ ID No. 13;
a mutant with a substitution of threonine (T) at position 274 of the amino acid sequence as shown in SEQ ID No. 1 to asparagine (N), a 4-amino acid truncation at the N-terminus of the amino acid sequence, and substitutions of arginine (R) at positions 475, 483 and 496 of the amino acid sequence to glycine (G), lysine (K) at positions 477, 497 and 501 to serine (S), lysine (K) at positions 482 and 498 to alanine (A), and lysine (K) at positions 495 and 500 to glycine (G), and the sequence of the mutant is as shown in SEQ ID No. 14.

In a further preferred embodiment of the human papillomavirus chimeric protein of the present invention, the polypeptide from the HPV type 73 L2 protein is any continuous fragment of 8-33 amino acids in the region of amino acid aa. 1-50 as shown in SEQ ID No. 2; preferably, the polypeptide is a RG-1 epitope peptide of the HPV type 73 L2 protein as shown in SEQ ID No. 2 or an epitope peptide of the mutant thereof; more preferably, the polypeptide is a polypeptide of amino acids 17 to 39 as shown in SEQ ID No. 2, or a mutant of the polypeptide of amino acids 17 to 39 as shown in SEQ ID No. 2 with 1- to 6-amino acid extension or truncation at the N-terminus and/or 1- to 6-amino acid extension or truncation at the C-terminus.

Preferably, the polypeptide from the HPV type 73 L2 protein is as shown in SEQ ID No. 15, SEQ ID No. 16 or SEQ ID No. 17.

Alternatively, the polypeptide from HPV type 73 L2 protein can further be a polypeptide with greater than 60%, preferably greater than 70%, greater than 80%, greater than 90%, and even more preferably greater than 95% sequence identity with the amino acid sequence as shown in SEQ ID No. 15, SEQ ID No. 16 or SEQ ID No. 17.

Alternatively, the polypeptide from the HPV type 73 L2 protein is inserted into the surface region of the HPV type 31 L1 protein or the mutant of the HPV type 31 L1 protein, preferably inserted into the DE loop or h4 region of the HPV type 31 L1 protein or the mutant of the HPV type 31 L1 protein, more preferably inserted between amino acids 132 and 133, or between amino acids 134 and 135, or between amino acids 136 and 137, or between amino acids 137 and 138, or between amino acids 432 and 433, or between amino acids 434 and 435, or between amino acids 435 and 436 of the HPV type 31 L1 protein or the mutant of the HPV type 31 L1 protein by direct insertion; alternatively inserted into the region of amino acids 132 to 136, or the region of amino acids 135 to 139, or the region of amino acids 428 to 431, or the region of amino acids 431 to 434 of the HPV type 31 L1 protein or the mutant of the HPV type 31 L1 protein by non-isometric substitution.

As used herein, the term "direct insertion" refers to the insertion of a selected peptide fragment between two adjacent amino acids. For example, direct insertion between amino acids 132 and 133 of SEQ ID No. 1 refers to the direct insertion of the selected peptide fragment between amino acids 132 and 133 of SEQ ID No. 1.

As used herein, the term "non-isometric substitution" refers to the insertion of a selected peptide fragment into the specified amino acid region after deleting the sequence of the specified amino acid region. For example, non-isometric substitution of the region of amino acids 132 to 136 of SEQ ID No. 1 refers to the insertion of the selected peptide fragment between amino acids 132 and 136 of SEQ ID No. 1 after deleting amino acids 133 to 135 of SEQ ID No. 1.

Optionally, in the embodiments of direct insertion or non-isometric substitution, the polypeptide from the HPV type 73 L2 protein comprises a linker of 1 to 3 amino acid residues in length at its N-terminus and/or C-terminus.

Optionally, the linker consists of any combination of amino acids selected from the group consisting of glycine (G), serine (S), alanine (A) and proline (P). Preferably, the linker at the N-terminus consists of G (glycine) P (proline), and the linker at the C-terminus consists of P (proline).

Alternatively, in an embodiment of the direct insertion, the amino acid sequence of the polypeptide from the HPV type 73 L2 protein is SEQ ID No. 15, SEQ ID No. 16 or SEQ ID No. 17, and the insertion site is between the amino acid 137 and amino acid 138 or between the amino acid 432 and amino acid 433 of the HPV type 31 L1 protein with a complete N-terminus and the mutant.

Alternatively, in an embodiment of the direct insertion, the amino acid sequence of the polypeptide from the HPV type 73 L2 protein is SEQ ID No. 15, SEQ ID No. 16 or SEQ ID No. 17, and the insertion site is between the amino acid 134 and amino acid 135 or between the amino acid 429 and amino acid 430 of the HPV type 31 L1 protein with a 4-amino acid truncation at the N-terminus and the mutant.

Alternatively, in an embodiment of the direct insertion, the amino acid sequence of the polypeptide from the HPV type 73 L2 protein is the sequence as shown in SEQ ID No. 15, SEQ ID No. 16 or SEQ ID No. 17 containing a GP linker at the N-terminus and/or a P linker at the C-terminus, and the insertion site is between the amino acid 137 and amino acid 138 or between the amino acid 432 and amino acid 433 of the HPV type 31 L1 protein with complete N-terminus and the mutant.

Alternatively, in an embodiment of the direct insertion, the amino acid sequence of the polypeptide from the HPV type 73 L2 protein is the sequence as shown in SEQ ID No. 15, SEQ ID No. 16 or SEQ ID No. 17 containing a GP linker at the N-terminus and/or a P linker at the C-terminus, and the insertion site is between the amino acid 134 and amino acid 135 or between the amino acid 429 and amino acid 430 of the HPV type 31 L1 protein with a 4-amino acid truncation at the N-terminus and the mutant.

Alternatively, in an embodiment of the non-isometric substitution, after deleting the region of amino acids 136-138 of the HPV type 31 L1 protein with complete N-terminus or the mutant, a polypeptide from the HPV type 73 L2 protein is inserted between the amino acids 135 and 139 of the HPV type 31 L1 protein with complete N-terminus or the mutant, the polypeptide from the HPV type 73 L2 protein has a glycine-proline linker added at its N-terminus, and the amino acid sequence of the polypeptide from the HPV type 73 L2 protein is as shown in SEQ ID No. 15, SEQ ID No. 16 or SEQ ID No. 17.

Alternatively, in an embodiment of the non-isometric substitution, after deleting the region of amino acids 133-135 of the HPV type 31 L1 protein with a 4-amino acid truncation at the N-terminus or the mutant, a polypeptide from the HPV type 73 L2 protein is inserted between the amino acids 132 and 136 of the HPV type 31 L1 protein with a 4-amino acid truncation at the N-terminus or the mutant, the polypeptide from the HPV type 73 L2 protein has a glycine-proline linker added at its N-terminus, and the amino acid sequence of the polypeptide from the HPV type 73 L2 protein is as shown in SEQ ID No. 15, SEQ ID No. 16 or SEQ ID No. 17.

Alternatively, in an embodiment of the non-isometric substitution, after deleting the region of amino acids 432-433 of the HPV type 31 L1 protein with complete N-terminus or the mutant, a polypeptide from the HPV type 73 L2 protein is inserted between the amino acids 431 and 434 of the HPV type 31 L1 protein with complete N-terminus or the mutant, and the amino acid sequence of the polypeptide from the HPV type 73 L2 protein is as shown in SEQ ID No. 15, SEQ ID No. 16 or SEQ ID No. 17.

Alternatively, in an embodiment of the non-isometric substitution, after deleting the region of amino acids 429-430 of the HPV type 31 L1 protein with a 4-amino acid truncation at the N-terminus or the mutant, a polypeptide from the HPV type 73 L2 protein is inserted between the amino acids 428 and 431 of the HPV type 31 L1 protein with a 4-amino acid truncation at the N-terminus or the mutant, and the amino acid sequence of the polypeptide from the HPV type 73 L2 protein is as shown in SEQ ID No. 15, SEQ ID No. 16 or SEQ ID No. 17.

Preferably, in the an embodiment of the non-isometric substitution, after deleting the region of amino acids 133-135 of the mutant of the HPV type 31 L1 protein, a polypeptide from the HPV type 73 L2 protein is inserted between the amino acids 132 and 136 of the mutant of the HPV type 31 L1 protein, the polypeptide from the HPV type 73 L2 protein has a glycine-proline linker added at its N-terminus, the amino acid sequence of the polypeptide from the HPV type 73 L2 protein is as shown in SEQ ID No. 15 or SEQ ID No. 17, and the amino acid sequence of the obtained chimeric protein is as shown in SEQ ID No. 18, SEQ ID No. 19, SEQ ID No. 20, SEQ ID No. 21, SEQ ID No. 22, SEQ ID No. 23, SEQ ID No. 24 or SEQ ID No. 25.

Preferably, in an embodiment of the non-isometric substitution, after deleting the region of amino acids 429-430 of the mutant of the HPV type 31 L1 protein, a polypeptide from the HPV type 73 L2 protein is inserted between the amino acids 428 and 431 of the mutant of the HPV type 31 L1 protein, the amino acid sequence of the polypeptide from the HPV type 73 L2 protein is as shown in SEQ ID No. 16 or SEQ ID No. 17, and the amino acid sequence of the obtained chimeric protein is as shown in SEQ ID No. 26, SEQ ID No. 27, SEQ ID No. 28, SEQ ID No. 29, SEQ ID No. 30, SEQ ID No. 31, SEQ ID No. 32 or SEQ ID No. 33.

In another aspect, the present invention relates to a polynucleotide encoding the above human papillomavirus chimeric protein.

The present invention also provides a vector comprising the above polynucleotide, as well as a cell comprising the vector.

The polynucleotide sequence encoding the above human papillomavirus chimeric protein of the present invention is suitable for different expression systems. Optionally, these nucleotide sequences are whole-gene optimized with *E. coli* codons and can be expressed at high levels in an *E. coli* expression system; alternatively, they are whole-gene optimized with insect cell codons and can be expressed at high levels in an insect cell expression system.

The present invention also provides a polymer, preferably, the polymer is a human papillomavirus chimeric pentamer or chimeric virus-like particle, wherein the polymer comprises or is formed by the human papillomavirus chimeric protein according to the present invention.

The present invention also provides use of the above papillomavirus chimeric protein, the papillomavirus chimeric pentamer or the above papillomavirus chimeric virus-like particle in the preparation of a vaccine for the prevention of papillomavirus infection and/or papillomavirus infection-induced diseases, preferably, the papillomavirus infection-induced diseases include, but are not limited to, cervical cancer, vaginal cancer, vulval cancer, penile cancer, perianal cancer, oropharyngeal cancer, tonsil cancer and oral cancer;
preferably, the papillomavirus infection is an infection selected from one or more of the following papillomavirus types: HPV16, HPV18, HPV26, HPV31, HPV33, HPV35, HPV39, HPV45, HPV51, HPV52, HPV53, HPV56, HPV58, HPV59, HPV66, HPV68, HPV70, HPV73; HPV6, HPV11, HPV2, HPV5, HPV27 and HPV57.

The present invention also provides a vaccine for the prevention of papillomavirus infection and infection-induced diseases, comprising the above papillomavirus chimeric pentamer or chimeric virus-like particle, an adjuvant, as well as an excipient or carrier for vaccines, preferably further comprising at least one virus-like particle or chimeric virus-like particle of HPV of the mucosa-tropic group and/or the skin-tropic group. Wherein, the content of these virus-like particles is an effective amount that can separately induce a protective immune response.

Alternatively, the adjuvant is an adjuvant for human use.

Description and explanation of relevant terms in the present invention

According to the present invention, the term "insect cell expression system" includes insect cell, recombinant baculovirus, recombinant Bacmid and expression vector. Among them, the insect cell is derived from commercially available cells, the examples of which are listed here but are not limited to: Sf9, Sf21, High Five.

According to the present invention, the term "prokaryotic expression system" includes but is not limited to *E. coli* expression system. Among them, the expression host bacteria are derived from commercially available strains, the examples of which are listed here but are not limited to: BL21 (DE3), BL21 (DE3) plysS, C43 (DE3), Rosetta-gami B (DE3).

According to the present invention, examples of the term "full-length HPV type 31 L1 protein" include, but are not limited to, full-length L1 protein with a length equal to the protein No. P17388.1, AEI61021.1, AEI60949.1, AAA92894.1, AIG59245.1, AIG59235.1 in the NCBI database.

The gene fragment of "truncated HPV type 31 L1 protein" means that it has deletion of nucleotides encoding 1 or more amino acids at its 5' end and/or 3' end compared to the gene of wild-type HPV type 31 L1 protein, wherein the full-length sequence of "wild-type HPV type 18 L1 protein" is shown in, for example, but not limited to, the following sequences in the NCBI database: P17388.1, AEI61021.1, AEI60949.1, AAA92894.1, AIG59245.1, AIG59235.1, etc.

According to the present invention, the term "excipient or carrier for vaccines" refers to that selected from one or more of the following, including but not limited to, pH adjuster, surfactant and ionic strength enhancer. For example, the pH adjuster is for example but not limited to phosphate buffer. The surfactant includes cationic, anionic or nonionic surfactant, and is for example but not limited to polysorbate 80 (Tween-80). The ionic strength enhancer is for example but not limited to sodium chloride.

According to the present invention, the term "adjuvant for human" refers to an adjuvant that can be applied clinically to the human body, including various adjuvants that have been approved and may be approved in the future, for example, but not limited to, aluminum adjuvant, MF59 and various forms of adjuvant compositions.

According to the present invention, the vaccine of the present invention can be in a patient-acceptable form, including but not limited to oral administration or injection, preferably injection.

According to the present invention, the vaccine of the present invention is preferably used in a unit dosage form, wherein the dose of protein virus-like particles in the unit dosage form is 5 µg to 100 µg, for example, 5, 10, 15, 20, 25, 30, 35, 40, 45, 50, 55, 60, 65, 70, 75, 80, 85, 90, 95, 100 µg, as well as the range between any two of the above values, preferably 30 µg to 60 µg.

### DESCRIPTION OF THE DRAWINGS

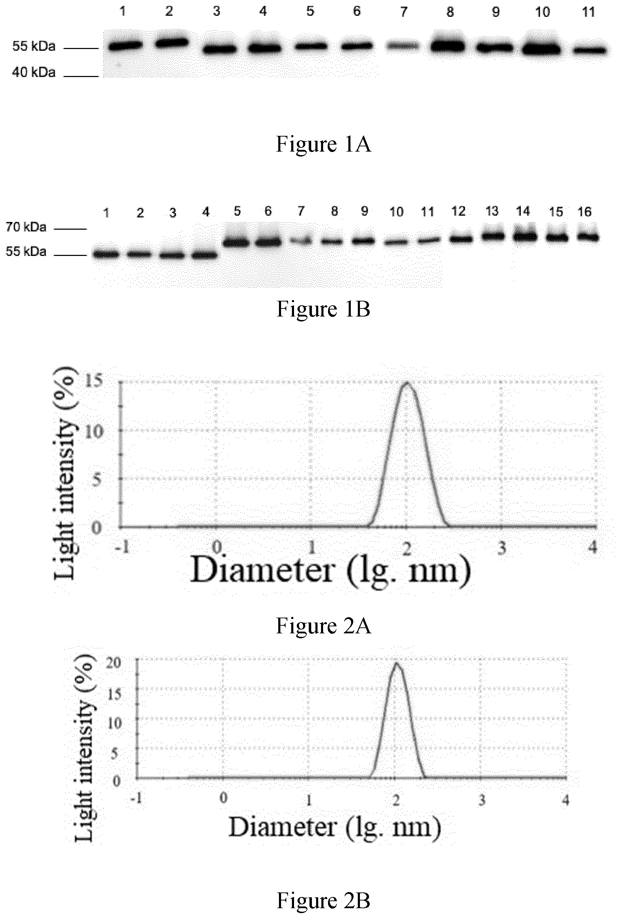

Figure 1A to Figure 1B: Identification of the expression of the mutants of type 31 L1 protein and chimeric proteins comprising same in Example 7 of the present invention in insect cells. The results showed that all of the 11 types of type 31 L1 protein and mutants and 16 types of chimeric proteins could be expressed in insect cells.
Figure 1A: Identification of the expression of type 31 L1 protein and mutant proteins thereof in insect cells: 1 represents 31L1; 2 represents T₂₇₄N; 3 represents 31L1MΔC; 4 represents T₂₇₄NΔC; 5 represents T₂₆₇AΔC; 6 represents T₂₆₇AT₂₇₄NΔC; 7 represents T₂₇₄NΔN2C; 8 represents T₂₇₄NΔN4C; 9 represents T₂₇₄NΔN5C; 10 represents T₂₇₄NΔN8C; 11 represents T₂₇₄NΔN10C;
Figure 1B: Identification of the expression of chimeric proteins comprising type 31 L1 protein mutants in insect cells: 1 represents 31L1DE₁₃₂₋₁₃₆/dE; 2 represents 31L1DE₁₃₂₋₁₃₆/dES; 3 represents 31L1h4₄₂₈₋₄₃₁/dE; 4 represents 31L1h4₄₂₈₋₄₃₁/dES; 5 represents 31L1DE₁₃₂₋₁₃₆/dE-CS1; 6 represents 31L1DE₁₃₂₋₁₃₆/dES-CS1; 7 represents 31L1h4₄₂₈₋₄₃₁/dE-CS1; 8 represents 31L1h4₄₂₈₋₄₃₁/dES-CS1; 9 represents 31L1DE₁₃₂₋₁₃₆/dE-CS2; 10 represents 31L1DE₁₃₂₋₁₃₆/dES-CS2; 11 represents 31L1h4₄₂₈₋₄₃₁/dE-CS2; 12 represents 31L1h4₄₂₈₋₄₃₁/dES-CS2; 13 represents 31L1DE₁₃₂₋₁₃₆/dE-CS3; 14 represents 31L1DE₁₃₂₋₁₃₆/dES-CS3; 15 represents 31L1h4₄₂₈₋₄₃₁/dE-CS3; and 16 represents 31L1h4₄₂₈₋₄₃₁/dES-CS3.

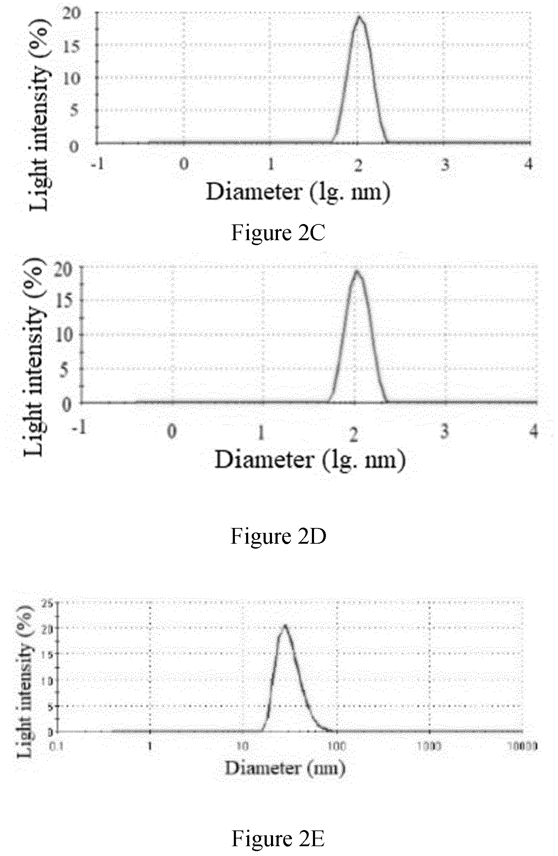

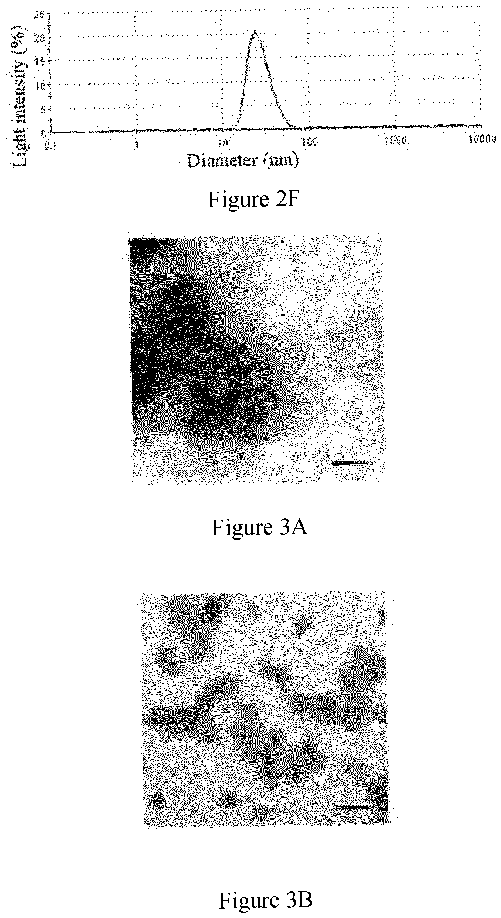

Figure 2A to Figure 2F: Results of dynamic light scattering analysis of VLPs and cVLPs obtained after purification in Example 8 of the present invention. The results showed that the hydraulic diameters of virus-like particles formed by 31L1MΔC, T₂₇₄NΔC, T₂₇₄NΔN4C, 31L1DE₁₃₂₋₁₃₆/dE, 31L1h4₄₂₈₋₄₃₁/dE and 31L1h4₄₂₈₋₄₃₁/dE-CS1 recombinant proteins were 103.3 nm, 99.78 nm, 106.8 nm, 104.59 nm, 47.8 nm and 42.4 nm, respectively, and the percentage of particle assembly were all 100%.
Figure 2A: 31L1MΔC; Figure 2B: T₂₇₄NΔC; Figure 2C: T₂₇₄NΔN4C; Figure 2D: 31L1DE₁₃₂₋₁₃₆/dE; Figure 2E: 31L1h4₄₂₈₋₄₃₁/dE; Figure 2F: 31L1h4₄₂₈₋₄₃₁/dE-CS1.
Figure 3A to Figure 3E: Results of transmission electron microscopy observation of VLPs and cVLPs obtained after purification in Example 8 of the present invention. A large number of virus-like particles could be seen in the field. Bar = 50 nm.

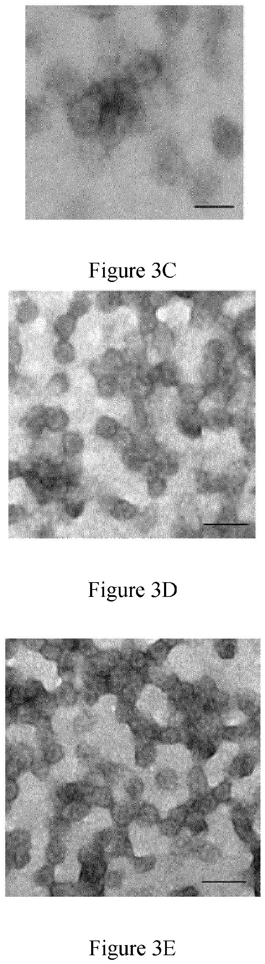

Figure 3A: 31L1MΔC; Figure 3B: T₂₇₄NΔN4C; Figure 3C: 31L1DE₁₃₂₋₁₃₆/dE; Figure 3D: 31L1h4₄₂₈₋₄₃₁/dE; Figure 3E: 31L1h4₄₂₈₋₄₃₁/dE-CS1.

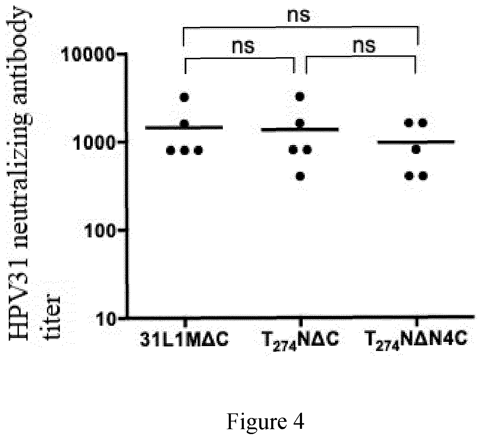

Figure 4: Results of detection of neutralizing antibody titers of the mouse immune serum according to Example 11 of the present invention using HPV31 pseudoviruses. ns: no statistical difference (*P* > 0.05).

### DETAILED DESCRIPTION OF THE INVENTION

The present invention will be further illustrated by the non-limiting examples below. It is well known to those skilled in the art that many modifications can be made to the present invention without departing from the spirit of the present invention, and such modifications also fall within the scope of the present invention. The following examples are only used to illustrate the present invention and should not be regarded as limiting the scope of the present invention, as the embodiments are necessarily diverse. The terms used in the present specification are intended only to describe particular embodiments but not as limitations. The scope of the present invention has been defined in the appended claims.

Unless otherwise specified, all the technical and scientific terms used in the present specification have the same meaning as those generally understood by those skilled in the technical field to which the present application relates. Preferred methods and materials of the present invention are described below, but any method and material similar or equivalent to the methods and materials described in the present specification can be used to implement or test the present invention. Unless otherwise specified, the following experimental methods are conventional methods or methods described in product specifications. Unless otherwise specified, the experimental materials used are easily available from commercial companies. All published literatures referred to in the present specification are incorporated here by reference to reveal and illustrate the methods and/or materials in the published literatures.

### Example 1: Sequence analysis of L1 of HPV31 variant strains

The keyword "major capsid protein L1 [Human papillomavirus type 31]" or "late protein L1 [Human papillomavirus type 31]" was entered into NCBI Genbank to obtain 19 variant strains of HPV31 L1 existing in nature, and the amino acid sequences were aligned using DNAMAN software (Table 1). It was found that the amino acids at positions 15, 179, 181, 194, 267, 274, 432, 439 of L1 were mutated, in which the positions 267 (mutation frequency 53%) and 274 (mutation frequency 89%) were high-frequency mutation sites, and the amino acid mutation frequencies of other sites were between 5%~15%. For the amino acids at positions 267 and 274, mutations from threonine (T) to alanine (A) at position 267 accounted for 53%, and mutations from threonine (T) to asparagine (N) at position 274 accounted for 89%. Therefore, A and T were the dominant amino acids at positions 267 and 274, respectively.

**Table 1. Alignment of amino acid sequences of different HPV31 L1 variant strains**

| 31L1 Sequence No. | Amino acid No. in L1 | | | | | | | |
|---|---|---|---|---|---|---|---|---|
| | 15 | 179 | 181 | 194 | 267 | 274 | 432 | 439 |
| P17388.1 (original) | P | N | I | T | T | T | T | E |
| AIG59271.1 | - | - | - | N | A | N | - | - |
| AIG59269.1 | - | - | L | - | A | N | - | - |
| AIG59267.1 | - | - | - | - | A | N | - | D |
| AIG59263.1 | - | - | - | - | A | N | - | - |
| AIG59261.1 | - | - | - | - | - | - | S | - |
| AIG59259.1 | - | - | - | N | - | N | - | - |
| AIG59257.1 | - | - | - | N | A | N | - | - |
| AIG59255.1 | - | - | - | - | A | N | - | - |
| AIG59253.1 | - | - | - | - | - | N | - | - |
| AIG59251.1 | - | - | - | - | - | - | S | - |
| AIG59249.1 | - | - | - | - | A | N | - | - |
| AIG59247.1 | - | - | - | - | - | N | - | - |
| AIG59245.1 | - | - | - | - | - | N | A | - |
| AIG59243.1 | - | - | - | - | - | N | - | - |
| AIG59241.1 | - | - | - | - | A | N | - | - |
| AIG59239.1 | - | - | - | - | - | N | - | - |
| AIG59237.1 | - | T | - | - | A | N | - | - |
| AIG59235.1 | N | - | - | - | - | N | - | - |
| AIG59233.1 | - | - | - | - | A | N | - | - |

| | | | | | | | | |
|---|---|---|---|---|---|---|---|---|
| ^{∗}The amino acids represented by hyphens (-) were the same as the amino acids in the corresponding positions of the original HPV31L1. | | | | | | | | |

### Example 2: Immunoactivity detection of different types of RG-1 epitope peptides

HPV35, -39, -51, -53, -56, -68, -73, -82 RG-1 epitope peptides were synthesized using chemical synthesis, and the sequences of the epitope peptides were as shown in Table 1. The polypeptides were synthesized by GL Biochem (Shanghai) Co., Ltd. In order to improve the immunogenicity of the synthetic peptides, each synthetic peptide was coupled with keyhole limpet hemocyanin (KLH) after activation of carboxyl group by 1-(3-dimethylaminopropyl)-3-ethylcarbodiimide hydrochloride (EDC, CAS No. 25952-53-8).

New Zealand white rabbits weighing 2.0-2.5 kg were randomly divided into groups, 2-4 rabbits per group. Four days before immunization, 15 mg of inactivated DH5a (PBS containing 0.5% v/v formaldehyde, treated at 37°C for 24-48 h) thoroughly mixed with an equal volume of Freund's complete adjuvant was injected subcutaneously at multiple sites on the back for immunostimulation. The first immunization was performed by subcutaneous injection of 1 mg of KLH-polypeptide thoroughly mixed with an equal volume of Freund's complete adjuvant at multiple sites on the back and inner thigh. Booster immunization was performed for 4 times at an interval of 2 weeks, and the antigen of the booster immunization was 0.5 mg of KLH-polypeptide thoroughly mixed with an equal volume of Freund's incomplete adjuvant. Blood was collected 2 weeks after the last immunization and serum was isolated.

17 types of HPV pseudoviruses were used to detect the titers of neutralizing antibodies in the immune serum, and the results were as shown in Table 3. The 73RG-1 epitope peptide had the best immune activity, and its antiserum could neutralize all 17 types used for detection, in which the titers of neutralizing antibodies of HPV45, -18, -16 were all above 10³, and the titers of neutralizing antibodies of HPV68, -57, -59, -39, -5 were between 500 and 1000.

Methods of polypeptide synthesis, pseudovirus preparation and pseudoviral neutralization experiments were all publicly available, for example, the patents CN 104418942A and 108676057A.

**Table 2. Sequences of different types of RG-1 epitope peptides synthesized**

| Type | Sequence of synthetic peptide | SEQ ID NO. |
|---|---|---|
| HPV35 | TQLYRTCKAAGTCPPDVIPKVEG | 53 |
| HPV39 | STLYRTCKQSGTCPPDVVDKVEG | 54 |
| HPV51 | TQLYSTCKAAGTCPPDVVNKVEG | 55 |
| HPV53 | TQLYQTCKQSGTCPEDVINKIEH | 56 |
| HPV56 | TQLYKTCKLSGTCPEDVVNKIEQ | 57 |
| HPV68 | STLYKTCKQSGTCPPDVINKVEG | 58 |
| HPV73 | TQLYKTCKQAGTCPPDVIPKVEG | 59 |
| HPV82 | TQLYSTCKAAGTCPPDVIPKVKG | 60 |

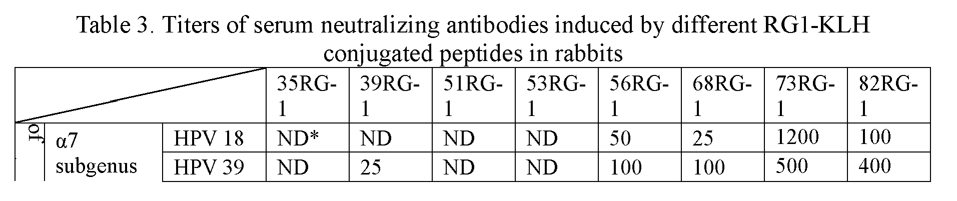

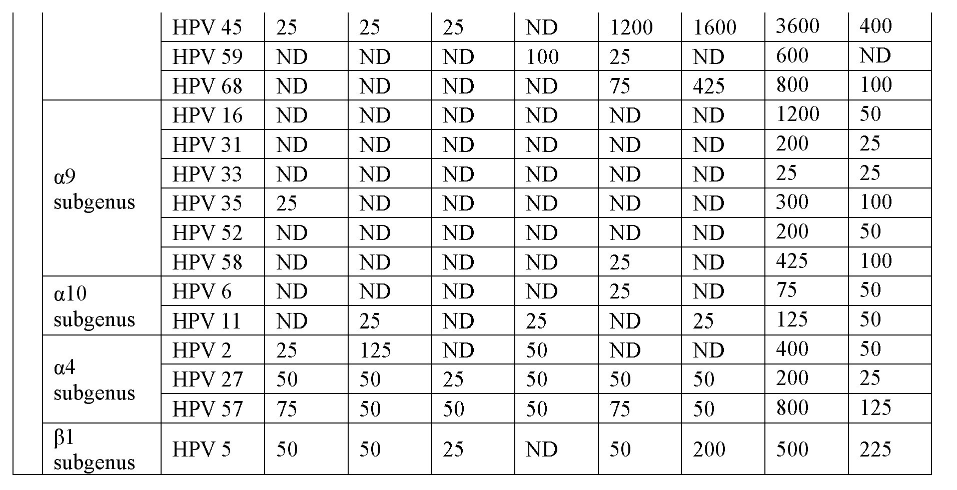

### Example 3: Synthesis of genes of the HPV31L1 protein and mutants thereof and construction of expression vectors

There were a total of 11 types of HPV31L1 protein and mutants, namely:
1) Original 31L1: its amino acid sequence was as shown in SEQ ID No. 1, and the nucleotide sequence encoding the 31L1 original protein was optimized with insect cell codons and constructed by whole-gene synthesis;
2) T₂₇₄N mutant: the threonine at position 274 of the sequence SEQ ID No. 1 was mutated to asparagine, its amino acid sequence was as shown in SEQ ID No. 3, and the nucleotide sequence encoding the T₂₇₄N mutant was optimized with insect cell codons and constructed by whole-gene synthesis;
3) 31L1MΔC mutant: 29 amino acids at the C-terminus of HPV31L1 were truncated, its amino acid sequence was as shown in SEQ ID No. 5, and the nucleotide sequence encoding 31L1MΔC was optimized with insect cell codons and constructed by whole-gene synthesis, the nucleotide sequence was as shown in SEQ ID No. 34;
4) T₂₇₄NΔC mutant: the threonine at position 274 of the sequence SEQ ID No. 5 was mutated to asparagine, its amino acid sequence was as shown in SEQ ID No. 6, and the nucleotide sequence encoding T₂₇₄NΔC was optimized with insect cell codons and constructed by whole-gene synthesis, the nucleotide sequence was as shown in SEQ ID No. 35;
5) T₂₆₇AΔC mutant: the threonine at position 267 of the sequence SEQ ID No. 5 was mutated to alanine, and the nucleotide sequence encoding T₂₆₇AΔC was optimized with insect cell codons and constructed by whole-gene synthesis;
6) T₂₆₇AT₂₇₄NΔC mutant: the threonine at position 267 of the sequence SEQ ID No. 6 was mutated to alanine, and the nucleotide sequence encoding T₂₆₇AT₂₇₄NΔC was optimized with insect cell codons and constructed by whole-gene synthesis;
7) T₂₇₄NΔN2C mutant: 2 amino acids at the N-terminus of the sequence as shown in SEQ ID No. 6 were truncated, its sequence was as shown in SEQ ID No. 7, and the nucleotide sequence encoding T₂₇₄NΔN2C was optimized with insect cell codons and constructed by whole-gene synthesis;
8) T₂₇₄NΔN4C mutant: 4 amino acids at the N-terminus of the sequence as shown in SEQ ID No. 6 were truncated, its amino acid sequence was as shown in SEQ ID No. 8, and the nucleotide sequence encoding T₂₇₄NΔN4C was optimized with insect cell codons and constructed by whole-gene synthesis, the nucleotide sequence was as shown in SEQ ID No. 36;
9) T₂₇₄NΔN5C mutant: 5 amino acids at the N-terminus of the sequence as shown in SEQ ID No. 6 were truncated, its sequence was as shown in SEQ ID No. 9, and the nucleotide sequence encoding T₂₇₄NΔN5C was optimized with insect cell codons and constructed by whole-gene synthesis;
10) T₂₇₄NΔN8C mutant: 8 amino acids at the N-terminus of the sequence as shown in SEQ ID No. 6 were truncated, its sequence was as shown in SEQ ID No. 10, and the nucleotide sequence encoding T₂₇₄NΔN8C was optimized with insect cell codons and constructed by whole-gene synthesis;
11) T₂₇₄NΔN10C mutant: 10 amino acids at the N-terminus of the sequence as shown in SEQ ID No. 6 were truncated, its sequence was as shown in SEQ ID No. 11, and the nucleotide sequence encoding T₂₇₄NΔN10C was optimized with insect cell codons and constructed by whole-gene synthesis.

The genes of HPV31L1 protein and mutants optimized with insect cell codons were digested by BamHI/Xbal and inserted into the commercial expression vector pFastBac1 (produced by Invitrogen), respectively. Expression vectors comprising the chimeric protein genes were obtained, namely pFastBac1-31L1, pFastBac1-T₂₇₄N, pFastBac1-31L1MΔC, pFastBac1-T₂₇₄NΔC, pFastBac1-T₂₆₇AΔC, pFastBac1-T₂₆₇AT₂₇₄NΔC, pFastBac1-T₂₇₄NΔN2C, pFastBac1-T₂₇₄NΔN4C, pFastBac1-T₂₇₄NΔN5C, pFastBac1-T₂₇₄NΔN8C, and pFastBac1-T₂₇₄NΔN10C. The above methods of enzymatic digestion, ligation and construction of clones were all well known, for example, the patent CN 101293918 B.

### Example 4: Synthesis of genes of the HPV31L1 chimeric protein and mutants thereof and construction of expression vectors

There were a total of 16 types of chimeric proteins and mutants, namely:
1) Chimeric L1 protein 31L1DE₁₃₂₋₁₃₆/dE: the backbone was T₂₇₄NΔN4C (i.e., 4 amino acids at the N-terminus were truncated and 29 amino acids at the C-terminus were truncated on the basis of mutation of threonine at position 274 to asparagine, its sequence was as shown in SEQ ID No. 8), where the region of aa. 133-135 was deleted, and the polypeptide of aa. 18-38 of HPV type 73 L2 protein comprising a GP linker at the N-terminus was fused between aa. 132/136 (inserted at the region of aa. 132-136 of SEQ ID No. 8 by non-isometric substitution). The amino acid sequence of the inserted fragment was the sequence as shown in SEQ ID No. 15 with glycine-proline added at the N-terminus, and the amino acid sequence of 31L1DE₁₃₂₋₁₃₆/dE chimeric protein was as shown in SEQ ID No. 18. The polynucleotide sequence encoding 31L1DE₁₃₂₋₁₃₆/dE was optimized with insect cell codons and constructed by whole-gene synthesis, and its sequence was as shown in SEQ ID No. 37;
2) Chimeric L1 protein 31L1DE₁₃₂₋₁₃₆/dES: the backbone was T₂₇₄NΔN4C (its sequence was as shown in SEQ ID No. 8), where the region of aa. 133-135 was deleted, and the polypeptide of aa. 19-35 of HPV type 73 L2 protein comprising a GP linker at the N-terminus was fused between aa. 132/136 (inserted at the region of aa. 132-136 of SEQ ID No. 8 by non-isometric substitution). The amino acid sequence of the inserted fragment was the sequence as shown in SEQ ID No. 17 with glycine-proline added at the N-terminus, and the amino acid sequence of 31L1DE₁₃₂₋₁₃₆/dES chimeric protein was as shown in SEQ ID No. 19. The polynucleotide sequence encoding 31L1DE₁₃₂₋₁₃₆/dES was optimized with insect cell codons and constructed by whole-gene synthesis, and its sequence was as shown in SEQ ID No. 38;
3) Chimeric L1 protein 31L1DE₁₃₂₋₁₃₆/dE-CS1: the backbone was T₂₇₄NΔN4C-CS1 (i.e., 4 amino acids at the N-terminus were truncated and the basic acids within 29 amino acids at the C-terminus were substituted on the basis of mutation of threonine at position 274 to asparagine, its sequence was as shown in SEQ ID No. 12), where the region of aa. 133-135 was deleted, and the polypeptide of aa. 18-38 of HPV type 73 L2 protein comprising a GP linker at the N-terminus was fused between aa. 132/136 (inserted at the region of aa. 132-136 of SEQ ID No. 12 by non-isometric substitution). The amino acid sequence of the inserted fragment was the sequence as shown in SEQ ID No. 15 with glycine-proline added at the N-terminus, and the amino acid sequence of 31L1DE₁₃₂₋₁₃₆/dE-CS1 chimeric protein was as shown in SEQ ID No. 20. The polynucleotide sequence encoding 31L1DE₁₃₂₋₁₃₆/dE-CS1 was optimized with insect cell codons and constructed by whole-gene synthesis, and its sequence was as shown in SEQ ID No. 39;
4) Chimeric L1 protein 31L1DE₁₃₂₋₁₃₆/dES-CS1: the backbone was T₂₇₄NΔN4C-CS1 (its sequence was as shown in SEQ ID No. 12), where the region of aa. 133-135 was deleted, and the polypeptide of aa. 19-35 of HPV type 73 L2 protein comprising a GP linker at the N-terminus was fused between aa. 132/136 (inserted at the region of aa. 132-136 of SEQ ID No. 12 by non-isometric substitution). The amino acid sequence of the inserted fragment was the sequence as shown in SEQ ID No. 17 with glycine-proline added at the N-terminus, and the amino acid sequence of 31L1DE₁₃₂₋₁₃₆/dES-CS1 chimeric protein was as shown in SEQ ID No. 21. The polynucleotide sequence encoding 31L1DE₁₃₂₋₁₃₆/dES-CS1 was optimized with insect cell codons and constructed by whole-gene synthesis, and its sequence was as shown in SEQ ID No. 40;
5) Chimeric L1 protein 31L1DE₁₃₂₋₁₃₆/dE-CS2: the backbone was T₂₇₄NΔN4C-CS2 (i.e., 4 amino acids at the N-terminus were truncated and the basic acids within 29 amino acids at the C-terminus were substituted on the basis of mutation of threonine at position 274 to asparagine, its sequence was as shown in SEQ ID No. 13), where the region of aa. 133-135 was deleted, and the polypeptide of aa. 18-38 of HPV type 73 L2 protein comprising a GP linker at the N-terminus was fused between aa. 132/136 (inserted at the region of aa. 132-136 of SEQ ID No. 13 by non-isometric substitution). The amino acid sequence of the inserted fragment was the sequence as shown in SEQ ID No. 15 with glycine-proline added at the N-terminus, and the amino acid sequence of 31L1DE₁₃₂₋₁₃₆/dE-CS2 chimeric protein was as shown in SEQ ID No. 22. The polynucleotide sequence encoding 31L1DE₁₃₂₋₁₃₆/dE-CS2 was optimized with insect cell codons and constructed by whole-gene synthesis, and its sequence was as shown in SEQ ID No. 41;
6) Chimeric L1 protein 31L1DE₁₃₂₋₁₃₆/dES-CS2: the backbone was T₂₇₄NΔN4C-CS2 (its sequence was as shown in SEQ ID No. 13), where the region of aa. 133-135 was deleted, and the polypeptide of aa. 19-35 of HPV type 73 L2 protein comprising a GP linker at the N-terminus was fused between aa. 132/136 (inserted at the region of aa. 132-136 of SEQ ID No. 13 by non-isometric substitution). The amino acid sequence of the inserted fragment was the sequence as shown in SEQ ID No. 17 with glycine-proline added at the N-terminus, and the amino acid sequence of 31L1DE₁₃₂₋₁₃₆/dES-CS2 chimeric protein was as shown in SEQ ID No. 23. The polynucleotide sequence encoding 31L1DE₁₃₂₋₁₃₆/dES-CS2 was optimized with insect cell codons and constructed by whole-gene synthesis, and its sequence was as shown in SEQ ID No. 42;
7) Chimeric L1 protein 31L1DE₁₃₂₋₁₃₆/dE-CS3: the backbone was T₂₇₄NΔN4C-CS3 (i.e., 4 amino acids at the N-terminus were truncated and the basic acids within 29 amino acids at the C-terminus were substituted on the basis of mutation of threonine at position 274 to asparagine, its sequence was as shown in SEQ ID No. 14), where the region of aa. 133-135 was deleted, and the polypeptide of aa. 18-38 of HPV type 73 L2 protein comprising a GP linker at the N-terminus was fused between aa. 132/136 (inserted at the region of aa. 132-136 of SEQ ID No. 14 by non-isometric substitution). The amino acid sequence of the inserted fragment was the sequence as shown in SEQ ID No. 15 with glycine-proline added at the N-terminus, and the amino acid sequence of 31L1DE₁₃₂₋₁₃₆/dE-CS3 chimeric protein was as shown in SEQ ID No. 24. The polynucleotide sequence encoding 31L1DE₁₃₂₋₁₃₆/dE-CS3 was optimized with insect cell codons and constructed by whole-gene synthesis, and its sequence was as shown in SEQ ID No. 43;
8) Chimeric L1 protein 31L1DE₁₃₂₋₁₃₆/dES-CS3: the backbone was T₂₇₄NΔN4C-CS3 (its sequence was as shown in SEQ ID No. 14), where the region of aa. 133-135 was deleted, and the polypeptide of aa. 19-35 of HPV type 73 L2 protein comprising a GP linker at the N-terminus was fused between aa. 132/136 (inserted at the region of aa. 132-136 of SEQ ID No. 14 by non-isometric substitution). The amino acid sequence of the inserted fragment was the sequence as shown in SEQ ID No. 17 with glycine-proline added at the N-terminus, and the amino acid sequence of 31L1DE₁₃₂₋₁₃₆/dES-CS3 chimeric protein was as shown in SEQ ID No. 25. The polynucleotide sequence encoding 31L1DE₁₃₂₋₁₃₆/dES-CS3 was optimized with insect cell codons and constructed by whole-gene synthesis, and its sequence was as shown in SEQ ID No. 44;
9) Chimeric L1 protein 31L1h4₄₂₈₋₄₃₁/dE: the backbone was T₂₇₄NΔN4C (i.e., 4 amino acids at the N-terminus were truncated and 29 amino acids at the C-terminus were truncated on the basis of mutation of threonine at position 274 to asparagine, its sequence was as shown in SEQ ID No. 8), where the region of aa. 429-430 was deleted, and the polypeptide of aa. 19-39 of HPV type 73 L2 protein was fused between aa. 428/431 (inserted at the region of aa. 428-431 of SEQ ID No. 8 by non-isometric substitution). The amino acid sequence of the inserted fragment was the sequence as shown in SEQ ID No. 16, and the amino acid sequence of 31L1h4₄₂₈₋₄₃₁/dE chimeric protein was as shown in SEQ ID No. 26. The polynucleotide sequence encoding 31L1h4₄₂₈₋₄₃₁/dE was optimized with insect cell codons and constructed by whole-gene synthesis, and its sequence was as shown in SEQ ID No. 45;
10) Chimeric L1 protein 31L1h4₄₂₈₋₄₃₁/dES: the backbone was T₂₇₄NΔN4C (its sequence was as shown in SEQ ID No. 8), where the region of aa. 429-430 was deleted, and the polypeptide of aa. 19-35 of HPV type 73 L2 protein was fused between aa. 428/431 (inserted at the region of aa. 428-431 of SEQ ID No. 8 by non-isometric substitution). The amino acid sequence of the inserted fragment was the sequence as shown in SEQ ID No. 17, and the amino acid sequence of 31L1h4₄₂₈₋₄₃₁/dES chimeric protein was as shown in SEQ ID No. 27. The polynucleotide sequence encoding 31L1h4₄₂₈₋₄₃₁/dES was optimized with insect cell codons and constructed by whole-gene synthesis, and its sequence was as shown in SEQ ID No. 46;
11) Chimeric L1 protein 31L1h4₄₂₈₋₄₃₁/dE-CS1: the backbone was T₂₇₄NΔN4C-CS1 (i.e., 4 amino acids at the N-terminus were truncated and the basic acids within 29 amino acids at the C-terminus were substituted on the basis of mutation of threonine at position 274 to asparagine, its sequence was as shown in SEQ ID No. 12), where the region of aa. 429-430 was deleted, and the polypeptide of aa. 19-39 of HPV type 73 L2 protein was fused between aa. 428/431 (inserted at the region of aa. 428-431 of SEQ ID No. 12 by non-isometric substitution). The amino acid sequence of the inserted fragment was the sequence as shown in SEQ ID No. 16, and the amino acid sequence of 31L1h4₄₂₈₋₄₃₁/dE-CS1 chimeric protein was as shown in SEQ ID No. 28. The polynucleotide sequence encoding 31L1h4₄₂₈₋₄₃₁/dE-CS1 was optimized with insect cell codons and constructed by whole-gene synthesis, and its sequence was as shown in SEQ ID No. 47;
12) Chimeric L1 protein 31L1h4₄₂₈₋₄₃₁/dES-CS1: the backbone was T₂₇₄NΔN4C-CS1 (its sequence was as shown in SEQ ID No. 12), where the region of aa. 429-430 was deleted, and the polypeptide of aa. 19-35 of HPV type 73 L2 protein was fused between aa. 428/431 (inserted at the region of aa. 428-431 of SEQ ID No. 12 by non-isometric substitution). The amino acid sequence of the inserted fragment was the sequence as shown in SEQ ID No. 17, and the amino acid sequence of 31L1h4₄₂₈₋₄₃₁/dES-CS1 chimeric protein was as shown in SEQ ID No. 29. The polynucleotide sequence encoding 31L1h4₄₂₈₋₄₃₁/dES-CS1 was optimized with insect cell codons and constructed by whole-gene synthesis, and its sequence was as shown in SEQ ID No. 48;
13) Chimeric L1 protein 31L1h4₄₂₈₋₄₃₁/dE-CS2: the backbone was T₂₇₄NΔN4C-CS2 (i.e., 4 amino acids at the N-terminus were truncated and the basic acids within 29 amino acids at the C-terminus were substituted on the basis of mutation of threonine at position 274 to asparagine, its sequence was as shown in SEQ ID No. 13), where the region of aa. 429-430 was deleted, and the polypeptide of aa. 19-39 of HPV type 73 L2 protein was fused between aa. 428/431 (inserted at the region of aa. 428-431 of SEQ ID No. 13 by non-isometric substitution). The amino acid sequence of the inserted fragment was the sequence as shown in SEQ ID No. 16, and the amino acid sequence of 31L1h4₄₂₈₋₄₃₁/dE-CS2 chimeric protein was as shown in SEQ ID No. 30. The polynucleotide sequence encoding 31L1h4₄₂₈₋₄₃₁/dE-CS2 was optimized with insect cell codons and constructed by whole-gene synthesis, and its sequence was as shown in SEQ ID No. 49;
14) Chimeric L1 protein 31L1h4₄₂₈₋₄₃₁/dES-CS2: the backbone was T₂₇₄NΔN4C-CS2 (its sequence was as shown in SEQ ID No. 13), where the region of aa. 429-430 was deleted, and the polypeptide of aa. 19-35 of HPV type 73 L2 protein was fused between aa. 428/431 (inserted at the region of aa. 428-431 of SEQ ID No. 13 by non-isometric substitution). The amino acid sequence of the inserted fragment was the sequence as shown in SEQ ID No. 17, and the amino acid sequence of 31L1h4₄₂₈₋₄₃₁/dES-CS2 chimeric protein was as shown in SEQ ID No. 31. The polynucleotide sequence encoding 31L1h4₄₂₈₋₄₃₁/dES-CS2 was optimized with insect cell codons and constructed by whole-gene synthesis, and its sequence was as shown in SEQ ID No. 50;
15) Chimeric L1 protein 31L1h4₄₂₈₋₄₃₁/dE-CS3: the backbone was T₂₇₄NΔN4C-CS3 (i.e., 4 amino acids at the N-terminus were truncated and the basic acids within 29 amino acids at the C-terminus were substituted on the basis of mutation of threonine at position 274 to asparagine, the sequence was as shown in SEQ ID No. 14), where the region of aa. 429-430 was deleted, and the polypeptide of aa. 19-39 of HPV type 73 L2 protein was fused between aa. 428/431 (inserted at the region of aa. 428-431 of SEQ ID No. 13 by non-isometric substitution). The amino acid sequence of the inserted fragment was the sequence as shown in SEQ ID No. 16, and the amino acid sequence of 31L1h4₄₂₈₋₄₃₁/dE-CS3 chimeric protein was as shown in SEQ ID No. 32. The polynucleotide sequence encoding 31L1h4₄₂₈₋₄₃₁/dE-CS3 was optimized with insect cell codons and constructed by whole-gene synthesis, and its sequence was as shown in SEQ ID No. 51;
16) Chimeric L1 protein 31L1h4₄₂₈₋₄₃₁/dES-CS3: the backbone was T₂₇₄NΔN4C-CS3 (its sequence was as shown in SEQ ID No. 14), where the region of aa. 429-430 was deleted, and the polypeptide of aa. 19-35 of HPV type 73 L2 protein was fused between aa. 428/431 (inserted at the region of aa. 428-431 of SEQ ID No. 14 by non-isometric substitution). The amino acid sequence of the inserted fragment was the sequence as shown in SEQ ID No. 17, and the amino acid sequence of 31L1h4₄₂₈₋₄₃₁/dES-CS3 chimeric protein was as shown in SEQ ID No. 33. The polynucleotide sequence encoding 31L1h4₄₂₈₋₄₃₁/dES-CS3 was optimized with insect cell codons and constructed by whole-gene synthesis, and its sequence was as shown in SEQ ID No. 52.

The genes of HPV31L1 protein and mutants optimized with insect cell codons were digested by BamHI/Xbal and inserted into the commercial expression vector pFastBac1 (produced by Invitrogen), respectively. Expression vectors comprising the chimeric protein genes were obtained, namely pFastBac1-31L1DE₁₃₂₋₁₃₆/dE, pFastBac1-31L1DE₁₃₂₋₁₃₆/dES, pFastBac1-31L1DE₁₃₂₋₁₃₆/dE-CS1, pFastBac1-31L1DE₁₃₂₋₁₃₆/dES-CS1, pFastBac1-31L1DE₁₃₂₋₁₃₆/dE-CS2, pFastBac1-31L1DE₁₃₂₋₁₃₆/dES-CS2, pFastBac1-31L1DE₁₃₂₋₁₃₆/dE-CS3, pFastBac1-31L1DE₁₃₂₋₁₃₆/dES-CS3, pFastBac1-31L1h4₄₂₈₋₄₃₁/dE, pFastBac1-31L1h4₄₂₈₋₄₃₁/dES, pFastBac1-31L1h4₄₂₈₋₄₃₁/dE-CS1, pFastBac1-31L1h4₄₂₈₋₄₃₁/dES-CS1, pFastBac1-31L1h4₄₂₈₋₄₃₁/dE-CS2, pFastBac1-31L1h4₄₂₈₋₄₃₁/dES-CS2, pFastBac1-31L1h4₄₂₈₋₄₃₁/dE-CS3, and pFastBac1-31L1h4₄₂₈₋₄₃₁/dES-CS3. The above methods of enzyme digestion, ligation and construction of clones were all well known, for example, the patent CN 101293918 B.

The amino acid sequences involved in the present invention were as described below:
HPV31L1 
MSLWRPSEAT VYLPPVPVSK VVSTDEYVTR TNIYYHAGSA RLLTVGHPYY SIPKSDNPKK
IVVPKVSGLQ YRVFRVRLPD PNKFGFPDTS FYNPETQRLV WACVGLEVGR GQPLGVGISG
HPLLNKFDDT ENSNRYAGGP GTDNRECISM DYKQTQLCLL GCKPPIGEHW GKGSPCSNNA
ITPGDCPPLE LKNSVIQDGD MVDTGFGAMD FTALQDTKSN VPLDICNSIC KYPDYLKMVA
EPYGDTLFFY LRREQMFVRH FFNRSGTVGE SVPTDLYIKG SGSTATLANS TYFPTPSGSM
VTSDAQIFNK PYWMQRAQGH NNGICWGNQL FVTVVDTTRS TNMSVCAAIA NSDTTFKSSN
 
FKEYLRHGEE FDLQFIFQLC KITLSADIMT YIHSMNPAIL EDWNFGLTTP PSGSLEDTYR
FVTSQAITCQ KTAPQKPKED PFKDYVFWEV NLKEKFSADL DQFPLGRKFL LQAGYRARPK
FKAGKRSAPS ASTTTPAKRK KTKK SEQ ID No.1
HPV73L2 
MRRKRDTHIR KKRASATQLY KTCKQAGTCP PDVIPKVEGS TIADNILKYG SIGVFFGGLG
IGSGSGSGGR TGYVPLSTGT PSKPVEMPLQ PIRPSVVTSV GPSDSSIVSL VEESSFIESG
IPGPTSIVPS TSGFDITTSV NSTPAIIDVS AISDTTQISV TTFKNPTFTD PSVLQPPPPL
EASGRLLFSN DTVTTHSYEN IPLDTFVVTT DHNSIVSSTP IPGRQPAARL GLYGRAIQQV
KVVDPAFLTT PTRLVTYDNP AFEGLQDTTL EFQHSDLHNA PDSDFLDIVK LHRPALTSRK
TGIRVSRLGQ RATLSTRSGK RIGAKVHFYH DISPIPTNDI EMQPLVTPQT PSIVTGSSIN
DGLYDVFLEN DVEDTVVQQT YTPTSIHSNS LVSSDVSTAT ANTTIPFSTG LDTHPGPDIA
LPLPSTETIF TPIVPLQPAG PIYIYGSGFI LHPSYYLLKR KRKRLSYSFT DVATY
SEQ ID No.2
T₂₇₄N 
MSLWRPSEAT VYLPPVPVSK VVSTDEYVTR TNIYYHAGSA RLLTVGHPYY SIPKSDNPKK
IVVPKVSGLQ YRVFRVRLPD PNKFGFPDTS FYNPETQRLV WACVGLEVGR GQPLGVGISG
HPLLNKFDDT ENSNRYAGGP GTDNRECISM DYKQTQLCLL GCKPPIGEHW GKGSPCSNNA
ITPGDCPPLE LKNSVIQDGD MVDTGFGAMD FTALQDTKSN VPLDICNSIC KYPDYLKMVA
EPYGDTLFFY LRREQMFVRH FFNRSGTVGE SVPNDLYIKG SGSTATLANS TYFPTPSGSM
VTSDAQIFNK PYWMQRAQGH NNGICWGNQL FVTVVDTTRS TNMSVCAAIA NSDTTFKSSN
FKEYLRHGEE FDLQFIFQLC KITLSADIMT YIHSMNPAIL EDWNFGLTTP PSGSLEDTYR
FVTSQAITCQ KTAPQKPKED PFKDYVFWEV NLKEKFSADL DQFPLGRKFL LQAGYRARPK
FKAGKRSAPS ASTTTPAKRK KTKK SEQ ID No.3
T₂₇₄NΔN4 
MRPSEAT VYLPPVPVSK VVSTDEYVTR TNIYYHAGSA RLLTVGHPYY SIPKSDNPKK
IVVPKVSGLQ YRVFRVRLPD PNKFGFPDTS FYNPETQRLV WACVGLEVGR GQPLGVGISG
HPLLNKFDDT ENSNRYAGGP GTDNRECISM DYKQTQLCLL GCKPPIGEHW GKGSPCSNNA
ITPGDCPPLE LKNSVIQDGD MVDTGFGAMD FTALQDTKSN VPLDICNSIC KYPDYLKMVA
EPYGDTLFFY LRREQMFVRH FFNRSGTVGE SVPNDLYIKG SGSTATLANS TYFPTPSGSM
VTSDAQIFNK PYWMQRAQGH NNGICWGNQL FVTVVDTTRS TNMSVCAAIA NSDTTFKSSN
FKEYLRHGEE FDLQFIFQLC KITLSADIMT YIHSMNPAIL EDWNFGLTTP PSGSLEDTYR
FVTSQAITCQ KTAPQKPKED PFKDYVFWEV NLKEKFSADL DQFPLGRKFL LQAGYRARPK
FKAGKRSAPS ASTTTPAKRK KTKK SEQ ID No.4
31L1ΔC29 
MSLWRPSEAT VYLPPVPVSK VVSTDEYVTR TNIYYHAGSA RLLTVGHPYY SIPKSDNPKK
IVVPKVSGLQ YRVFRVRLPD PNKFGFPDTS FYNPETQRLV WACVGLEVGR GQPLGVGISG
HPLLNKFDDT ENSNRYAGGP GTDNRECISM DYKQTQLCLL GCKPPIGEHW GKGSPCSNNA
ITPGDCPPLE LKNSVIQDGD MVDTGFGAMD FTALQDTKSN VPLDICNSIC KYPDYLKMVA
EPYGDTLFFY LRREQMFVRH FFNRSGTVGE SVPTDLYIKG SGSTATLANS TYFPTPSGSM
VTSDAQIFNK PYWMQRAQGH NNGICWGNQL FVTVVDTTRS TNMSVCAAIA NSDTTFKSSN
FKEYLRHGEE FDLQFIFQLC KITLSADIMT YIHSMNPAIL EDWNFGLTTP PSGSLEDTYR
FVTSQAITCQ KTAPQKPKED PFKDYVFWEV NLKEKFSADL DQFPLGRKFL LQAGY
SEQ ID No.5
T₂₇₄NΔC29 
MSLWRPSEAT VYLPPVPVSK VVSTDEYVTR TNIYYHAGSA RLLTVGHPYY SIPKSDNPKK
IVVPKVSGLQ YRVFRVRLPD PNKFGFPDTS FYNPETQRLV WACVGLEVGR GQPLGVGISG
HPLLNKFDDT ENSNRYAGGP GTDNRECISM DYKQTQLCLL GCKPPIGEHW GKGSPCSNNA
ITPGDCPPLE LKNSVIQDGD MVDTGFGAMD FTALQDTKSN VPLDICNSIC KYPDYLKMVA
EPYGDTLFFY LRREQMFVRH FFNRSGTVGE SVPNDLYIKG SGSTATLANS TYFPTPSGSM
VTSDAQIFNK PYWMQRAQGH NNGICWGNQL FVTVVDTTRS TNMSVCAAIA NSDTTFKSSN
FKEYLRHGEE FDLQFIFQLC KITLSADIMT YIHSMNPAIL EDWNFGLTTP PSGSLEDTYR
FVTSQAITCQ KTAPQKPKED PFKDYVFWEV NLKEKFSADL DQFPLGRKFL LQAGY
SEQ ID No.6
T₂₇₄NΔN2C29 
MLWRPSEAT VYLPPVPVSK VVSTDEYVTR TNIYYHAGSA RLLTVGHPYY SIPKSDNPKK
IVVPKVSGLQ YRVFRVRLPD PNKFGFPDTS FYNPETQRLV WACVGLEVGR GQPLGVGISG
HPLLNKFDDT ENSNRYAGGP GTDNRECISM DYKQTQLCLL GCKPPIGEHW GKGSPCSNNA
ITPGDCPPLE LKNSVIQDGD MVDTGFGAMD FTALQDTKSN VPLDICNSIC KYPDYLKMVA
EPYGDTLFFY LRREQMFVRH FFNRSGTVGE SVPNDLYIKG SGSTATLANS TYFPTPSGSM
VTSDAQIFNK PYWMQRAQGH NNGICWGNQL FVTVVDTTRS TNMSVCAAIA NSDTTFKSSN
FKEYLRHGEE FDLQFIFQLC KITLSADIMT YIHSMNPAIL EDWNFGLTTP PSGSLEDTYR
FVTSQAITCQ KTAPQKPKED PFKDYVFWEV NLKEKFSADL DQFPLGRKFL LQAGY
SEQ ID No.7
T₂₇₄NΔN4C29 
MRPSEAT VYLPPVPVSK VVSTDEYVTR TNIYYHAGSA RLLTVGHPYY SIPKSDNPKK
IVVPKVSGLQ YRVFRVRLPD PNKFGFPDTS FYNPETQRLV WACVGLEVGR GQPLGVGISG
HPLLNKFDDT ENSNRYAGGP GTDNRECISM DYKQTQLCLL GCKPPIGEHW GKGSPCSNNA
ITPGDCPPLE LKNSVIQDGD MVDTGFGAMD FTALQDTKSN VPLDICNSIC KYPDYLKMVA
EPYGDTLFFY LRREQMFVRH FFNRSGTVGE SVPNDLYIKG SGSTATLANS TYFPTPSGSM
VTSDAQIFNK PYWMQRAQGH NNGICWGNQL FVTVVDTTRS TNMSVCAAIA NSDTTFKSSN
FKEYLRHGEE FDLQFIFQLC KITLSADIMT YIHSMNPAIL EDWNFGLTTP PSGSLEDTYR
FVTSQAITCQ KTAPQKPKED PFKDYVFWEV NLKEKFSADL DQFPLGRKFL LQAGY
SEQ ID No.8
T₂₇₄NΔN5C29 
MPSEAT VYLPPVPVSK VVSTDEYVTR TNIYYHAGSA RLLTVGHPYY SIPKSDNPKK
IVVPKVSGLQ YRVFRVRLPD PNKFGFPDTS FYNPETQRLV WACVGLEVGR GQPLGVGISG
HPLLNKFDDT ENSNRYAGGP GTDNRECISM DYKQTQLCLL GCKPPIGEHW GKGSPCSNNA
ITPGDCPPLE LKNSVIQDGD MVDTGFGAMD FTALQDTKSN VPLDICNSIC KYPDYLKMVA
EPYGDTLFFY LRREQMFVRH FFNRSGTVGE SVPNDLYIKG SGSTATLANS TYFPTPSGSM
VTSDAQIFNK PYWMQRAQGH NNGICWGNQL FVTVVDTTRS TNMSVCAAIA NSDTTFKSSN
FKEYLRHGEE FDLQFIFQLC KITLSADIMT YIHSMNPAIL EDWNFGLTTP PSGSLEDTYR
FVTSQAITCQ KTAPQKPKED PFKDYVFWEV NLKEKFSADL DQFPLGRKFL LQAGY
SEQ ID No.9
T₂₇₄NΔN8C29 MAT VYLPPVPVSK VVSTDEYVTR TNIYYHAGSA RLLTVGHPYY SIPKSDNPKK 
IVVPKVSGLQ YRVFRVRLPD PNKFGFPDTS FYNPETQRLV WACVGLEVGR GQPLGVGISG
HPLLNKFDDT ENSNRYAGGP GTDNRECISM DYKQTQLCLL GCKPPIGEHW GKGSPCSNNA
ITPGDCPPLE LKNSVIQDGD MVDTGFGAMD FTALQDTKSN VPLDICNSIC KYPDYLKMVA
EPYGDTLFFY LRREQMFVRH FFNRSGTVGE SVPNDLYIKG SGSTATLANS TYFPTPSGSM
VTSDAQIFNK PYWMQRAQGH NNGICWGNQL FVTVVDTTRS TNMSVCAAIA NSDTTFKSSN
FKEYLRHGEE FDLQFIFQLC KITLSADIMT YIHSMNPAIL EDWNFGLTTP PSGSLEDTYR
FVTSQAITCQ KTAPQKPKED PFKDYVFWEV NLKEKFSADL DQFPLGRKFL LQAGY
SEQ ID No.10
T₂₇₄NΔN10C29 
MVYLPPVPVSK VVSTDEYVTR TNIYYHAGSA RLLTVGHPYY SIPKSDNPKK
IVVPKVSGLQ YRVFRVRLPD PNKFGFPDTS FYNPETQRLV WACVGLEVGR GQPLGVGISG
HPLLNKFDDT ENSNRYAGGP GTDNRECISM DYKQTQLCLL GCKPPIGEHW GKGSPCSNNA
ITPGDCPPLE LKNSVIQDGD MVDTGFGAMD FTALQDTKSN VPLDICNSIC KYPDYLKMVA
EPYGDTLFFY LRREQMFVRH FFNRSGTVGE SVPNDLYIKG SGSTATLANS TYFPTPSGSM
VTSDAQIFNK PYWMQRAQGH NNGICWGNQL FVTVVDTTRS TNMSVCAAIA NSDTTFKSSN
FKEYLRHGEE FDLQFIFQLC KITLSADIMT YIHSMNPAIL EDWNFGLTTP PSGSLEDTYR
FVTSQAITCQ KTAPQKPKED PFKDYVFWEV NLKEKFSADL DQFPLGRKFL LQAGY
SEQ ID No.11
T₂₇₄N-CS1 
MRPSEATVYL PPVPVSKVVS TDEYVTRTNI YYHAGSARLL TVGHPYYSIP KSDNPKKIVV
PKVSGLQYRV FRVRLPDPNK FGFPDTSFYN PETQRLVWAC VGLEVGRGQP LGVGISGHPL
LNKFDDTENS NRYAGGPGTD NRECISMDYK QTQLCLLGCK PPIGEHWGKG SPCSNNAITP
GDCPPLELKN SVIQDGDMVD TGFGAMDFTA LQDTKSNVPL DICNSICKYP DYLKMVAEPY
GDTLFFYLRR EQMFVRHFFN RSGTVGESVP NDLYIKGSGS TATLANSTYF PTPSGSMVTS
DAQIFNKPYW MQRAQGHNNG ICWGNQLFVT VVDTTRSTNM SVCAAIANSD TTFKSSNFKE
YLRHGEEFDL QFIFQLCKIT LSADIMTYIH SMNPAILEDW NFGLTTPPSG SLEDTYRFVT
SQAITCQKTA PQKPKEDPFK DYVFWEVNLK EKFSADLDQF PLGRKFLLQA GYRAGPSFAA
GAGSAPSAST TTPAGGSATG S

SEQ ID No.12
T₂₇₄N-CS2 
MRPSEATVYL PPVPVSKVVS TDEYVTRTNI YYHAGSARLL TVGHPYYSIP KSDNPKKIVV
PKVSGLQYRV FRVRLPDPNK FGFPDTSFYN PETQRLVWAC VGLEVGRGQP LGVGISGHPL
LNKFDDTENS NRYAGGPGTD NRECISMDYK QTQLCLLGCK PPIGEHWGKG SPCSNNAITP
GDCPPLELKN SVIQDGDMVD TGFGAMDFTA LQDTKSNVPL DICNSICKYP DYLKMVAEPY
GDTLFFYLRR EQMFVRHFFN RSGTVGESVP NDLYIKGSGS TATLANSTYF PTPSGSMVTS
DAQIFNKPYW MQRAQGHNNG ICWGNQLFVT VVDTTRSTNM SVCAAIANSD TTFKSSNFKE
YLRHGEEFDL QFIFQLCKIT LSADIMTYIH SMNPAILEDW NFGLTTPPSG SLEDTYRFVT
SQAITCQKTA PQKPKEDPFK DYVFWEVNLK EKFSADLDQF PLGRKFLLQA GYGAGPSFAA
GAGSAPSAST TTPAGGSATG S
SEQ ID No.13
T₂₇₄N-CS3 
MRPSEATVYL PPVPVSKVVS TDEYVTRTNI YYHAGSARLL TVGHPYYSIP KSDNPKKIVV
PKVSGLQYRV FRVRLPDPNK FGFPDTSFYN PETQRLVWAC VGLEVGRGQP LGVGISGHPL
LNKFDDTENS NRYAGGPGTD NRECISMDYK QTQLCLLGCK PPIGEHWGKG SPCSNNAITP
73L2 aa.18-38

| | |
|---|---|
| QLYKTCKQAGTCPPDVIPKVE | SEQ ID No.15 |

73L2 aa.19-39

| | |
|---|---|
| LYKTCKQAGTCPPDVIPKVEG | SEQ ID No.16 |

73L2 aa.19-35

| | |
|---|---|
| LYKTCKQAGTCPPDVIP | SEQ ID No.17 |

31L1DE₁₃₂₋₁₃₆/dE 
MRPSEATVYL PPVPVSKVVS TDEYVTRTNI YYHAGSARLL TVGHPYYSIP KSDNPKKIVV
PKVSGLQYRV FRVRLPDPNK FGFPDTSFYN PETQRLVWAC VGLEVGRGQP LGVGISGHPL
LNKFDDTENS NRGPQLYKTC KQAGTCPPDV IPKVEGPGTD NRECISMDYK QTQLCLLGCK
PPIGEHWGKG SPCSNNAITP GDCPPLELKN SVIQDGDMVD TGFGAMDFTA LQDTKSNVPL
DICNSICKYP DYLKMVAEPY GDTLFFYLRR EQMFVRHFFN RSGTVGESVP NDLYIKGSGS
TATLANSTYF PTPSGSMVTS DAQIFNKPYW MQRAQGHNNG ICWGNQLFVT VVDTTRSTNM
SVCAAIANSD TTFKSSNFKE YLRHGEEFDL QFIFQLCKIT LSADIMTYIH SMNPAILEDW
NFGLTTPPSG SLEDTYRFVT SQAITCQKTA PQKPKEDPFK DYVFWEVNLK EKFSADLDQF
PLGRKFLLQA GY SEQ ID No.18
31L1DE₁₃₂₋₁₃₆/dES 
MRPSEATVYL PPVPVSKVVS TDEYVTRTNI YYHAGSARLL TVGHPYYSIP KSDNPKKIVV
PKVSGLQYRV FRVRLPDPNK FGFPDTSFYN PETQRLVWAC VGLEVGRGQP LGVGISGHPL
LNKFDDTENS NRGPLYKTCK QAGTCPPDVI PGPGTDNREC ISMDYKQTQL CLLGCKPPIG
EHWGKGSPCS NNAITPGDCP PLELKNSVIQ DGDMVDTGFG AMDFTALQDT KSNVPLDICN
SICKYPDYLK MVAEPYGDTL FFYLRREQMF VRHFFNRSGT VGESVPNDLY IKGSGSTATL
ANSTYFPTPS GSMVTSDAQI FNKPYWMQRA QGHNNGICWG NQLFVTVVDT TRSTNMSVCA
AIANSDTTFK SSNFKEYLRH GEEFDLQFIF QLCKITLSAD IMTYIHSMNP AILEDWNFGL
TTPPSGSLED TYRFVTSQAI TCQKTAPQKP KEDPFKDYVF WEVNLKEKFS ADLDQFPLGR
KFLLQAGY SEQ ID No.19
31L1DE₁₃₂₋₁₃₆/dE-CS1
31L1DE₁₃₂₋₁₃₆/dES-CS1
31L1DE₁₃₂₋₁₃₆/dE-CS2
31L1DE₁₃₂₋₁₃₆/dES-CS2
31L1DE₁₃₂₋₁₃₆/dE-CS3
31L1DE₁₃₂₋₁₃₆/dES-CS3
31L1h4₄₂₈₋₄₃₁/dE 
MRPSEATVYL PPVPVSKVVS TDEYVTRTNI YYHAGSARLL TVGHPYYSIP KSDNPKKIVV
PKVSGLQYRV FRVRLPDPNK FGFPDTSFYN PETQRLVWAC VGLEVGRGQP LGVGISGHPL
LNKFDDTENS NRYAGGPGTD NRECISMDYK QTQLCLLGCK PPIGEHWGKG SPCSNNAITP
GDCPPLELKN SVIQDGDMVD TGFGAMDFTA LQDTKSNVPL DICNSICKYP DYLKMVAEPY
GDTLFFYLRR EQMFVRHFFN RSGTVGESVP NDLYIKGSGS TATLANSTYF PTPSGSMVTS
DAQIFNKPYW MQRAQGHNNG ICWGNQLFVT VVDTTRSTNM SVCAAIANSD TTFKSSNFKE
YLRHGEEFDL QFIFQLCKIT LSADIMTYIH SMNPAILEDW NFGLTTPPSG SLEDTYRFVT
SQAITCQKLY KTCKQAGTCP PDVIPKVEGP QKPKEDPFKD YVFWEVNLKE KFSADLDQFP
LGRKFLLQAG Y SEQ ID No.26
31L1h4₄₂₈₋₄₃₁/dES 
MRPSEATVYL PPVPVSKVVS TDEYVTRTNI YYHAGSARLL TVGHPYYSIP KSDNPKKIVV
PKVSGLQYRV FRVRLPDPNK FGFPDTSFYN PETQRLVWAC VGLEVGRGQP LGVGISGHPL
LNKFDDTENS NRYAGGPGTD NRECISMDYK QTQLCLLGCK PPIGEHWGKG SPCSNNAITP
GDCPPLELKN SVIQDGDMVD TGFGAMDFTA LQDTKSNVPL DICNSICKYP DYLKMVAEPY
GDTLFFYLRR EQMFVRHFFN RSGTVGESVP NDLYIKGSGS TATLANSTYF PTPSGSMVTS
DAQIFNKPYW MQRAQGHNNG ICWGNQLFVT VVDTTRSTNM SVCAAIANSD TTFKSSNFKE
YLRHGEEFDL QFIFQLCKIT LSADIMTYIH SMNPAILEDW NFGLTTPPSG SLEDTYRFVT
SQAITCQKLY KTCKQAGTCP PDVIPPQKPK EDPFKDYVFW EVNLKEKFSA DLDQFPLGRK
FLLQAGY SEQ ID No.27
31L1h4₄₂₈₋₄₃₁/dE-CS1
31L1h4₄₂₈₋₄₃₁/dES-CS1
31L1h4₄₂₈₋₄₃₁/dE-CS2
31L1h4₄₂₈₋₄₃₁/dES-CS2
31L1h4₄₂₈₋₄₃₁/dE-CS3
31L1h4₄₂₈₋₄₃₁/dES-CS3
31L1ΔC29 nt 
atgagcctgt ggagaccatc agaggctaca gtatatctgc cacctgttcc tgtaagcaaa
gtggtttcaa ccgatgagta cgtaacacgt accaacatct actatcacgc tggatctgcg
cgcctcctga ctgtcggtca cccatactac tctattccca agtcagacaa tcccaagaaa
atcgtggtac ccaaagtgag cggactccag tatcgtgttt tcagagtccg cttgccagat
cccaacaagt ttggcttccc agacacaagc ttctacaatc ctgaaaccca acgcctggta
tgggcatgcg tgggactcga ggttggccgt ggtcagcctc tgggagtggg catctcaggc
cacccattgc tcaacaaatt cgatgacacc gagaattcca acagatacgc gggtggacca
ggtacagata accgcgaatg catcagcatg gactacaagc agacccaact gtgcctcttg
ggctgcaagc caccaattgg agagcactgg ggcaaaggct caccttgctc caacaacgct
atcacacctg gagactgccc acccttggaa ctcaagaatt ctgtcattca ggatggtgac
atggtggaca ctggctttgg tgcaatggat ttcaccgctc ttcaagacac caagtcaaac
gtacctctgg atatctgcaa tagcatttgc aagtatccag actacctcaa gatggttgct
 gagccttacg gtgatacact gttcttctac ctgagacgtg agcagatgtt tgtgagacac
ttcttcaacc gttccggcac tgtcggagag tcagttccta cagacctcta catcaagggt
tctggcagca cagcaactct ggcgaactca acctactttc ctactccttc cggatctatg
gtcacgagcg atgctcagat cttcaacaag ccctactgga tgcaacgtgc ccagggacac
aacaatggca tttgctgggg caatcagctc ttcgtcactg ttgtggacac tactcgctcc
actaacatgt ctgtctgcgc tgccattgcc aactccgata ccactttcaa aagctctaac
tttaaggaat atctgcgtca cggtgaggag ttcgacttgc agttcatctt ccaactctgc
aagatcaccc tgtccgctga tatcatgacc tacattcaca gcatgaatcc agctatcctg
gaagactgga acttcggtct gaccactcca ccctctggta gcctggagga tacctacagg
tttgttacat ctcaagcaat cacttgccag aagactgccc cacagaagcc taaagaggac
cccttcaaag attacgtctt ctgggaggtg aatctgaagg agaagttctc tgctgatttg
gatcagtttc cactgggtcg taagttcctg ctccaagctg gatactaag
SEQ ID No.34
T₂₇₄NΔC29 nt 
atgagcctgt ggagaccatc agaggctaca gtatatctgc cacctgttcc tgtaagcaaa
gtggtttcaa ccgatgagta cgtaacacgt accaacatct actatcacgc tggatctgcg
cgcctcctga ctgtcggtca cccatactac tctattccca agtcagacaa tcccaagaaa
atcgtggtac ccaaagtgag cggactccag tatcgtgttt tcagagtccg cttgccagat
cccaacaagt ttggcttccc agacacaagc ttctacaatc ctgaaaccca acgcctggta
tgggcatgcg tgggactcga ggttggccgt ggtcagcctc tgggagtggg catctcaggc
cacccattgc tcaacaaatt cgatgacacc gagaattcca acagatacgc gggtggacca
ggtacagata accgcgaatg catcagcatg gactacaagc agacccaact gtgcctcttg
ggctgcaagc caccaattgg agagcactgg ggcaaaggct caccttgctc caacaacgct
atcacacctg gagactgccc acccttggaa ctcaagaatt ctgtcattca ggatggtgac
atggtggaca ctggctttgg tgcaatggat ttcaccgctc ttcaagacac caagtcaaac
gtacctctgg atatctgcaa tagcatttgc aagtatccag actacctcaa gatggttgct
 
gagccttacg gtgatacact gttcttctac ctgagacgtg agcagatgtt tgtgagacac
ttcttcaacc gttccggcac tgtcggagag tcagttccta acgacctcta catcaagggt
tctggcagca cagcaactct ggcgaactca acctactttc ctactccttc cggatctatg
gtcacgagcg atgctcagat cttcaacaag ccctactgga tgcaacgtgc ccagggacac
aacaatggca tttgctgggg caatcagctc ttcgtcactg ttgtggacac tactcgctcc
actaacatgt ctgtctgcgc tgccattgcc aactccgata ccactttcaa aagctctaac
tttaaggaat atctgcgtca cggtgaggag ttcgacttgc agttcatctt ccaactctgc
aagatcaccc tgtccgctga tatcatgacc tacattcaca gcatgaatcc agctatcctg
gaagactgga acttcggtct gaccactcca ccctctggta gcctggagga tacctacagg
tttgttacat ctcaagcaat cacttgccag aagactgccc cacagaagcc taaagaggac
cccttcaaag attacgtctt ctgggaggtg aatctgaagg agaagttctc tgctgatttg
gatcagtttc cactgggtcg taagttcctg ctccaagctg gatactaag
SEQ ID No.35
T₂₇₄NΔN4C29 
atgagaccat cagaggctac agtatatctg ccacctgttc ctgtaagcaa agtggtttca
accgatgagt acgtaacacg taccaacatc tactatcacg ctggatctgc gcgcctcctg
actgtcggtc acccatacta ctctattccc aagtcagaca atcccaagaa aatcgtggta
cccaaagtga gcggactcca gtatcgtgtt ttcagagtcc gcttgccaga tcccaacaag
tttggcttcc cagacacaag cttctacaat cctgaaaccc aacgcctggt atgggcatgc
gtgggactcg aggttggccg tggtcagcct ctgggagtgg gcatctcagg ccacccattg
ctcaacaaat tcgatgacac cgagaattcc aacagatacg cgggtggacc aggtacagat
aaccgcgaat gcatcagcat ggactacaag cagacccaac tgtgcctctt gggctgcaag
ccaccaattg gagagcactg gggcaaaggc tcaccttgct ccaacaacgc tatcacacct
ggagactgcc cacccttgga actcaagaat tctgtcattc aggatggtga catggtggac
actggctttg gtgcaatgga tttcaccgct cttcaagaca ccaagtcaaa cgtacctctg
gatatctgca atagcatttg caagtatcca gactacctca agatggttgc tgagccttac
ggtgatacac tgttcttcta cctgagacgt gagcagatgt ttgtgagaca cttcttcaac
cgttccggca ctgtcggaga gtcagttcct aacgacctct acatcaaggg ttctggcagc
acagcaactc tggcgaactc aacctacttt cctactcctt ccggatctat ggtcacgagc
gatgctcaga tcttcaacaa gccctactgg atgcaacgtg cccagggaca caacaatggc
atttgctggg gcaatcagct cttcgtcact gttgtggaca ctactcgctc cactaacatg
tctgtctgcg ctgccattgc caactccgat accactttca aaagctctaa ctttaaggaa
tatctgcgtc acggtgagga gttcgacttg cagttcatct tccaactctg caagatcacc
ctgtccgctg atatcatgac ctacattcac agcatgaatc cagctatcct ggaagactgg
aacttcggtc tgaccactcc accctctggt agcctggagg atacctacag gtttgttaca
tctcaagcaa tcacttgcca gaagactgcc ccacagaagc ctaaagagga ccccttcaaa
gattacgtct tctgggaggt gaatctgaag gagaagttct ctgctgattt ggatcagttt
ccactgggtc gtaagttcct gctccaagct ggatactaag

SEQ ID No.36
31L1DE₁₃₂₋₁₃₆/dE nt 
atgagaccat cagaggctac agtatatctg ccacctgttc ctgtaagcaa agtggtttca
accgatgagt acgtaacacg taccaacatc tactatcacg ctggatctgc gcgcctcctg
actgtcggtc acccatacta ctctattccc aagtcagaca atcccaagaa aatcgtggta
cccaaagtga gcggactcca gtatcgtgtt ttcagagtcc gcttgccaga tcccaacaag
tttggcttcc cagacacaag cttctacaat cctgaaaccc aacgcctggt atgggcatgc
 
gtgggactcg aggttggccg tggtcagcct ctgggagtgg gcatctcagg ccacccattg
ctcaacaaat tcgatgacac cgagaattcc aacagaggtc ctcagctgta caagacctgc
aagcaggctg gtacctgccc tcctgacgtg atccctaagg tggagggacc aggtacagat
aaccgcgaat gcatcagcat ggactacaag cagacccaac tgtgcctctt gggctgcaag
ccaccaattg gagagcactg gggcaaaggc tcaccttgct ccaacaacgc tatcacacct
ggagactgcc cacccttgga actcaagaat tctgtcattc aggatggtga catggtggac
actggctttg gtgcaatgga tttcaccgct cttcaagaca ccaagtcaaa cgtacctctg
gatatctgca atagcatttg caagtatcca gactacctca agatggttgc tgagccttac
ggtgatacac tgttcttcta cctgagacgt gagcagatgt ttgtgagaca cttcttcaac
cgttccggca ctgtcggaga gtcagttcct aacgacctct acatcaaggg ttctggcagc
acagcaactc tggcgaactc aacctacttt cctactcctt ccggatctat ggtcacgagc
gatgctcaga tcttcaacaa gccctactgg atgcaacgtg cccagggaca caacaatggc
atttgctggg gcaatcagct cttcgtcact gttgtggaca ctactcgctc cactaacatg
tctgtctgcg ctgccattgc caactccgat accactttca aaagctctaa ctttaaggaa
tatctgcgtc acggtgagga gttcgacttg cagttcatct tccaactctg caagatcacc
ctgtccgctg atatcatgac ctacattcac agcatgaatc cagctatcct ggaagactgg
aacttcggtc tgaccactcc accctctggt agcctggagg atacctacag gtttgttaca
tctcaagcaa tcacttgcca gaagactgcc ccacagaagc ctaaagagga ccccttcaaa
gattacgtct tctgggaggt gaatctgaag gagaagttct ctgctgattt ggatcagttt
ccactgggtc gtaagttcct gctccaagct ggatactaag

SEQ ID No.37
31L1DE₁₃₂₋₁₃₆/dES nt 
atgagaccat cagaggctac agtatatctg ccacctgttc ctgtaagcaa agtggtttca
accgatgagt acgtaacacg taccaacatc tactatcacg ctggatctgc gcgcctcctg
actgtcggtc acccatacta ctctattccc aagtcagaca atcccaagaa aatcgtggta
cccaaagtga gcggactcca gtatcgtgtt ttcagagtcc gcttgccaga tcccaacaag
tttggcttcc cagacacaag cttctacaat cctgaaaccc aacgcctggt atgggcatgc
gtgggactcg aggttggccg tggtcagcct ctgggagtgg gcatctcagg ccacccattg
ctcaacaaat tcgatgacac cgagaattcc aacagaggtc ctctgtacaa gacctgcaag
caggctggta cctgccctcc tgacgtgatc cctggaccag gtacagataa ccgcgaatgc
atcagcatgg actacaagca gacccaactg tgcctcttgg gctgcaagcc accaattgga
gagcactggg gcaaaggctc accttgctcc aacaacgcta tcacacctgg agactgccca
cccttggaac tcaagaattc tgtcattcag gatggtgaca tggtggacac tggctttggt
gcaatggatt tcaccgctct tcaagacacc aagtcaaacg tacctctgga tatctgcaat
agcatttgca agtatccaga ctacctcaag atggttgctg agccttacgg tgatacactg
ttcttctacc tgagacgtga gcagatgttt gtgagacact tcttcaaccg ttccggcact
gtcggagagt cagttcctaa cgacctctac atcaagggtt ctggcagcac agcaactctg
gcgaactcaa cctactttcc tactccttcc ggatctatgg tcacgagcga tgctcagatc
ttcaacaagc cctactggat gcaacgtgcc cagggacaca acaatggcat ttgctggggc
aatcagctct tcgtcactgt tgtggacact actcgctcca ctaacatgtc tgtctgcgct
gccattgcca actccgatac cactttcaaa agctctaact ttaaggaata tctgcgtcac
ggtgaggagt tcgacttgca gttcatcttc caactctgca agatcaccct gtccgctgat
atcatgacct acattcacag catgaatcca gctatcctgg aagactggaa cttcggtctg
accactccac cctctggtag cctggaggat acctacaggt ttgttacatc tcaagcaatc
acttgccaga agactgcccc acagaagcct aaagaggacc ccttcaaaga ttacgtcttc
tgggaggtga atctgaagga gaagttctct gctgatttgg atcagtttcc actgggtcgt
 aagttcctgc tccaagctgg atactaag SEQ ID No.38
31L1DE₁₃₂₋₁₃₆/dE-CS1 nt 
atgagaccat cagaggctac agtatatctg ccacctgttc ctgtaagcaa agtggtttca
accgatgagt acgtaacacg taccaacatc tactatcacg ctggatctgc gcgcctcctg
actgtcggtc acccatacta ctctattccc aagtcagaca atcccaagaa aatcgtggta
cccaaagtga gcggactcca gtatcgtgtt ttcagagtcc gcttgccaga tcccaacaag
tttggcttcc cagacacaag cttctacaat cctgaaaccc aacgcctggt atgggcatgc
gtgggactcg aggttggccg tggtcagcct ctgggagtgg gcatctcagg ccacccattg
ctcaacaaat tcgatgacac cgagaattcc aacagaggtc ctcagctgta caagacctgc
aagcaggctg gtacctgccc tcctgacgtg atccctaagg tggagggacc aggtacagat
aaccgcgaat gcatcagcat ggactacaag cagacccaac tgtgcctctt gggctgcaag
ccaccaattg gagagcactg gggcaaaggc tcaccttgct ccaacaacgc tatcacacct
ggagactgcc cacccttgga actcaagaat tctgtcattc aggatggtga catggtggac
actggctttg gtgcaatgga tttcaccgct cttcaagaca ccaagtcaaa cgtacctctg
gatatctgca atagcatttg caagtatcca gactacctca agatggttgc tgagccttac
ggtgatacac tgttcttcta cctgagacgt gagcagatgt ttgtgagaca cttcttcaac
cgttccggca ctgtcggaga gtcagttcct aacgacctct acatcaaggg ttctggcagc
acagcaactc tggcgaactc aacctacttt cctactcctt ccggatctat ggtcacgagc
gatgctcaga tcttcaacaa gccctactgg atgcaacgtg cccagggaca caacaatggc
atttgctggg gcaatcagct cttcgtcact gttgtggaca ctactcgctc cactaacatg
tctgtctgcg ctgccattgc caactccgat accactttca aaagctctaa ctttaaggaa
tatctgcgtc acggtgagga gttcgacttg cagttcatct tccaactctg caagatcacc
ctgtccgctg atatcatgac ctacattcac agcatgaatc cagctatcct ggaagactgg
aacttcggtc tgaccactcc accctctggt agcctggagg atacctacag gtttgttaca
tctcaagcaa tcacttgcca gaagactgcc ccacagaagc ctaaagagga ccccttcaaa
gattacgtct tctgggaggt gaatctgaag gagaagttct ctgctgattt ggatcagttt
ccactgggtc gtaagttcct gctccaagct ggataccgtg ctggtccttc gtttgccgct
ggcgcgggtt cggctcctag cgcctcgact accacgccgg ctggcggttc ggccacgggc
agctaag
SEQ ID No.39
31L1DE₁₃₂₋₁₃₆/dES-CS1 nt 
atgagaccat cagaggctac agtatatctg ccacctgttc ctgtaagcaa agtggtttca
accgatgagt acgtaacacg taccaacatc tactatcacg ctggatctgc gcgcctcctg
actgtcggtc acccatacta ctctattccc aagtcagaca atcccaagaa aatcgtggta
cccaaagtga gcggactcca gtatcgtgtt ttcagagtcc gcttgccaga tcccaacaag
tttggcttcc cagacacaag cttctacaat cctgaaaccc aacgcctggt atgggcatgc
gtgggactcg aggttggccg tggtcagcct ctgggagtgg gcatctcagg ccacccattg
ctcaacaaat tcgatgacac cgagaattcc aacagaggtc ctctgtacaa gacctgcaag
caggctggta cctgccctcc tgacgtgatc cctggaccag gtacagataa ccgcgaatgc
atcagcatgg actacaagca gacccaactg tgcctcttgg gctgcaagcc accaattgga
gagcactggg gcaaaggctc accttgctcc aacaacgcta tcacacctgg agactgccca
cccttggaac tcaagaattc tgtcattcag gatggtgaca tggtggacac tggctttggt
gcaatggatt tcaccgctct tcaagacacc aagtcaaacg tacctctgga tatctgcaat
agcatttgca agtatccaga ctacctcaag atggttgctg agccttacgg tgatacactg
ttcttctacc tgagacgtga gcagatgttt gtgagacact tcttcaaccg ttccggcact
 
gtcggagagt cagttcctaa cgacctctac atcaagggtt ctggcagcac agcaactctg
gcgaactcaa cctactttcc tactccttcc ggatctatgg tcacgagcga tgctcagatc
ttcaacaagc cctactggat gcaacgtgcc cagggacaca acaatggcat ttgctggggc
aatcagctct tcgtcactgt tgtggacact actcgctcca ctaacatgtc tgtctgcgct
gccattgcca actccgatac cactttcaaa agctctaact ttaaggaata tctgcgtcac
ggtgaggagt tcgacttgca gttcatcttc caactctgca agatcaccct gtccgctgat
atcatgacct acattcacag catgaatcca gctatcctgg aagactggaa cttcggtctg
accactccac cctctggtag cctggaggat acctacaggt ttgttacatc tcaagcaatc
acttgccaga agactgcccc acagaagcct aaagaggacc ccttcaaaga ttacgtcttc
tgggaggtga atctgaagga gaagttctct gctgatttgg atcagtttcc actgggtcgt
aagttcctgc tccaagctgg ataccgtg ctggtccttc gtttgccgct
ggcgcgggtt cggctcctag cgcctcgact accacgccgg ctggcggttc ggccacgggc
agctaag
SEQ ID No.40
31L1DE₁₃₂₋₁₃₆/dE-CS2 nt 
atgagaccat cagaggctac agtatatctg ccacctgttc ctgtaagcaa agtggtttca
accgatgagt acgtaacacg taccaacatc tactatcacg ctggatctgc gcgcctcctg
actgtcggtc acccatacta ctctattccc aagtcagaca atcccaagaa aatcgtggta
cccaaagtga gcggactcca gtatcgtgtt ttcagagtcc gcttgccaga tcccaacaag
tttggcttcc cagacacaag cttctacaat cctgaaaccc aacgcctggt atgggcatgc
gtgggactcg aggttggccg tggtcagcct ctgggagtgg gcatctcagg ccacccattg
ctcaacaaat tcgatgacac cgagaattcc aacagaggtc ctcagctgta caagacctgc
aagcaggctg gtacctgccc tcctgacgtg atccctaagg tggagggacc aggtacagat
aaccgcgaat gcatcagcat ggactacaag cagacccaac tgtgcctctt gggctgcaag
ccaccaattg gagagcactg gggcaaaggc tcaccttgct ccaacaacgc tatcacacct
ggagactgcc caccettgga actcaagaat tctgtcattc aggatggtga catggtggac
actggctttg gtgcaatgga tttcaccgct cttcaagaca ccaagtcaaa cgtacctctg
gatatctgca atagcatttg caagtatcca gactacctca agatggttgc tgagccttac
ggtgatacac tgttcttcta cctgagacgt gagcagatgt ttgtgagaca cttcttcaac
cgttccggca ctgtcggaga gtcagttcct aacgacctct acatcaaggg ttctggcagc
acagcaactc tggcgaactc aacctacttt cctactcctt ccggatctat ggtcacgagc
gatgctcaga tcttcaacaa gccctactgg atgcaacgtg cccagggaca caacaatggc
atttgctggg gcaatcagct cttcgtcact gttgtggaca ctactcgctc cactaacatg
tctgtctgcg ctgccattgc caactccgat accactttca aaagctctaa ctttaaggaa
tatctgcgtc acggtgagga gttcgacttg cagttcatct tccaactctg caagatcacc
ctgtccgctg atatcatgac ctacattcac agcatgaatc cagctatcct ggaagactgg
aacttcggtc tgaccactcc accctctggt agcctggagg atacctacag gtttgttaca
tctcaagcaa tcacttgcca gaagactgcc ccacagaagc ctaaagagga ccccttcaaa
gattacgtct tctgggaggt gaatctgaag gagaagttct ctgctgattt ggatcagttt
ccactgggtc gtaagttcct gctccaagct ggatacggcg ctggtccttc gtttgccgct
ggcgcgggtt cggctcctag cgcctcgact accacgccgg ctggcggttc ggccacgggc
agctaag
SEQ ID No.41
31L1DE₁₃₂₋₁₃₆/dES-CS2 nt atgagaccat cagaggctac agtatatctg ccacctgttc ctgtaagcaa agtggtttca 
accgatgagt acgtaacacg taccaacatc tactatcacg ctggatctgc gcgcctcctg
actgtcggtc acccatacta ctctattccc aagtcagaca atcccaagaa aatcgtggta
cccaaagtga gcggactcca gtatcgtgtt ttcagagtcc gcttgccaga tcccaacaag
tttggcttcc cagacacaag cttctacaat cctgaaaccc aacgcctggt atgggcatgc
gtgggactcg aggttggccg tggtcagcct ctgggagtgg gcatctcagg ccacccattg
ctcaacaaat tcgatgacac cgagaattcc aacagaggtc ctctgtacaa gacctgcaag
caggctggta cctgccctcc tgacgtgatc cctggaccag gtacagataa ccgcgaatgc
atcagcatgg actacaagca gacccaactg tgcctcttgg gctgcaagcc accaattgga
gagcactggg gcaaaggctc accttgctcc aacaacgcta tcacacctgg agactgccca
cccttggaac tcaagaattc tgtcattcag gatggtgaca tggtggacac tggctttggt
gcaatggatt tcaccgctct tcaagacacc aagtcaaacg tacctctgga tatctgcaat
agcatttgca agtatccaga ctacctcaag atggttgctg agccttacgg tgatacactg
ttcttctacc tgagacgtga gcagatgttt gtgagacact tcttcaaccg ttccggcact
gtcggagagt cagttcctaa cgacctctac atcaagggtt ctggcagcac agcaactctg
gcgaactcaa cctactttcc tactccttcc ggatctatgg tcacgagcga tgctcagatc
ttcaacaagc cctactggat gcaacgtgcc cagggacaca acaatggcat ttgctggggc
aatcagctct tcgtcactgt tgtggacact actcgctcca ctaacatgtc tgtctgcgct
gccattgcca actccgatac cactttcaaa agctctaact ttaaggaata tctgcgtcac
ggtgaggagt tcgacttgca gttcatcttc caactctgca agatcaccct gtccgctgat
atcatgacct acattcacag catgaatcca gctatcctgg aagactggaa cttcggtctg
accactccac cctctggtag cctggaggat acctacaggt ttgttacatc tcaagcaatc
acttgccaga agactgcccc acagaagcct aaagaggacc ccttcaaaga ttacgtcttc
tgggaggtga atctgaagga gaagttctct gctgatttgg atcagtttcc actgggtcgt
aagttcctgc tccaagctgg atacggcg ctggtccttc gtttgccgct
ggcgcgggtt cggctcctag cgcctcgact accacgccgg ctggcggttc ggccacgggc
agctaag
SEQ ID No.42
31L1DE₁₃₂₋₁₃₆/dE-CS3 nt 
atgagaccat cagaggctac agtatatctg ccacctgttc ctgtaagcaa agtggtttca
accgatgagt acgtaacacg taccaacatc tactatcacg ctggatctgc gcgcctcctg
actgtcggtc acccatacta ctctattccc aagtcagaca atcccaagaa aatcgtggta
cccaaagtga gcggactcca gtatcgtgtt ttcagagtcc gcttgccaga tcccaacaag
tttggcttcc cagacacaag cttctacaat cctgaaaccc aacgcctggt atgggcatgc
gtgggactcg aggttggccg tggtcagcct ctgggagtgg gcatctcagg ccacccattg
ctcaacaaat tcgatgacac cgagaattcc aacagaggtc ctcagctgta caagacctgc
aagcaggctg gtacctgccc tcctgacgtg atccctaagg tggagggacc aggtacagat
aaccgcgaat gcatcagcat ggactacaag cagacccaac tgtgcctctt gggctgcaag
ccaccaattg gagagcactg gggcaaaggc tcaccttgct ccaacaacgc tatcacacct
ggagactgcc cacccttgga actcaagaat tctgtcattc aggatggtga catggtggac
actggctttg gtgcaatgga tttcaccgct cttcaagaca ccaagtcaaa cgtacctctg
gatatctgca atagcatttg caagtatcca gactacctca agatggttgc tgagccttac
ggtgatacac tgttcttcta cctgagacgt gagcagatgt ttgtgagaca cttcttcaac
cgttccggca ctgtcggaga gtcagttcct aacgacctct acatcaaggg ttctggcagc
acagcaactc tggcgaactc aacctacttt cctactcctt ccggatctat ggtcacgagc
gatgctcaga tcttcaacaa gccctactgg atgcaacgtg cccagggaca caacaatggc
atttgctggg gcaatcagct cttcgtcact gttgtggaca ctactcgctc cactaacatg
 
tctgtctgcg ctgccattgc caactccgat accactttca aaagctctaa ctttaaggaa
tatctgcgtc acggtgagga gttcgacttg cagttcatct tccaactctg caagatcacc
ctgtccgctg atatcatgac ctacattcac agcatgaatc cagctatcct ggaagactgg
aacttcggtc tgaccactcc accctctggt agcctggagg atacctacag gtttgttaca
tctcaagcaa tcacttgcca gaagactgcc ccacagaagc ctaaagagga ccccttcaaa
gattacgtct tctgggaggt gaatctgaag gagaagttct ctgctgattt ggatcagttt
ccactgggtc gtaagttcct gctccaagct ggataccgtg ctggtccttc gtttaaagc
tggcgcgggt tcggctccta gcgcctcgac taccacgccg gctggcggtt cggccacggg
cagctaag

SEQ ID No.43
31L1DE₁₃₂₋₁₃₆/dES-CS3 nt 
atgagaccat cagaggctac agtatatctg ccacctgttc ctgtaagcaa agtggtttca
accgatgagt acgtaacacg taccaacatc tactatcacg ctggatctgc gcgcctcctg
actgtcggtc acccatacta ctctattccc aagtcagaca atcccaagaa aatcgtggta
cccaaagtga gcggactcca gtatcgtgtt ttcagagtcc gcttgccaga tcccaacaag
tttggcttcc cagacacaag cttctacaat cctgaaaccc aacgcctggt atgggcatgc
gtgggactcg aggttggccg tggtcagcct ctgggagtgg gcatctcagg ccacccattg
ctcaacaaat tcgatgacac cgagaattcc aacagaggtc ctctgtacaa gacctgcaag
caggctggta cctgccctcc tgacgtgatc cctggaccag gtacagataa ccgcgaatgc
atcagcatgg actacaagca gacccaactg tgcctcttgg gctgcaagcc accaattgga
gagcactggg gcaaaggctc accttgctcc aacaacgcta tcacacctgg agactgccca
cccttggaac tcaagaattc tgtcattcag gatggtgaca tggtggacac tggctttggt
gcaatggatt tcaccgctct tcaagacacc aagtcaaacg tacctctgga tatctgcaat
agcatttgca agtatccaga ctacctcaag atggttgctg agccttacgg tgatacactg
ttcttctacc tgagacgtga gcagatgttt gtgagacact tcttcaaccg ttccggcact
gtcggagagt cagttcctaa cgacctctac atcaagggtt ctggcagcac agcaactctg
gcgaactcaa cctactttcc tactccttcc ggatctatgg tcacgagcga tgctcagatc
ttcaacaagc cctactggat gcaacgtgcc cagggacaca acaatggcat ttgctggggc
aatcagctct tcgtcactgt tgtggacact actcgctcca ctaacatgtc tgtctgcgct
gccattgcca actccgatac cactttcaaa agctctaact ttaaggaata tctgcgtcac
ggtgaggagt tcgacttgca gttcatcttc caactctgca agatcaccct gtccgctgat
atcatgacct acattcacag catgaatcca gctatcctgg aagactggaa cttcggtctg
accactccac cctctggtag cctggaggat acctacaggt ttgttacatc tcaagcaatc
acttgccaga agactgcccc acagaagcct aaagaggacc ccttcaaaga ttacgtcttc
tgggaggtga atctgaagga gaagttctct gctgatttgg atcagtttcc actgggtcgt
aagttcctgc tccaagctgg ataccgtg ctggtccttc gtttaaagc
tggcgcgggt tcggctccta gcgcctcgac taccacgccg gctggcggtt cggccacggg
cagctaag
SEQ ID No.44
31L1h4₄₂₈₋₄₃₁/dE nt 
atgagaccat cagaggctac agtatatctg ccacctgttc ctgtaagcaa agtggtttca
accgatgagt acgtaacacg taccaacatc tactatcacg ctggatctgc gcgcctcctg
actgtcggtc acccatacta ctctattccc aagtcagaca atcccaagaa aatcgtggta
cccaaagtga gcggactcca gtatcgtgtt ttcagagtcc gcttgccaga tcccaacaag
tttggcttcc cagacacaag cttctacaat cctgaaaccc aacgcctggt atgggcatgc
 
gtgggactcg aggttggccg tggtcagcct ctgggagtgg gcatctcagg ccacccattg
ctcaacaaat tcgatgacac cgagaattcc aacagatacg cgggtggacc aggtacagat
aaccgcgaat gcatcagcat ggactacaag cagacccaac tgtgcctctt gggctgcaag
ccaccaattg gagagcactg gggcaaaggc tcaccttgct ccaacaacgc tatcacacct
ggagactgcc cacccttgga actcaagaat tctgtcattc aggatggtga catggtggac
actggctttg gtgcaatgga tttcaccgct cttcaagaca ccaagtcaaa cgtacctctg
gatatctgca atagcatttg caagtatcca gactacctca agatggttgc tgagccttac
ggtgatacac tgttcttcta cctgagacgt gagcagatgt ttgtgagaca cttcttcaac
cgttccggca ctgtcggaga gtcagttcct aacgacctct acatcaaggg ttctggcagc
acagcaactc tggcgaactc aacctacttt cctactcctt ccggatctat ggtcacgagc
gatgctcaga tcttcaacaa gccctactgg atgcaacgtg cccagggaca caacaatggc
atttgctggg gcaatcagct cttcgtcact gttgtggaca ctactcgctc cactaacatg
tctgtctgcg ctgccattgc caactccgat accactttca aaagctctaa ctttaaggaa
tatctgcgtc acggtgagga gttcgacttg cagttcatct tccaactctg caagatcacc
ctgtccgctg atatcatgac ctacattcac agcatgaatc cagctatcct ggaagactgg
aacttcggtc tgaccactcc accctctggt agcctggagg atacctacag gtttgttaca
tctcaagcaa tcacttgcca gaagctgtac aagacctgca agcaggctgg tacctgccct
cctgacgtga tccctaaggt ggagggtcca cagaagccta aagaggaccc cttcaaagat
tacgtcttct gggaggtgaa tctgaaggag aagttctctg ctgatttgga tcagtttcca
ctgggtcgta agttcctgct ccaagctgga tactaag

SEQ ID No.45
31L1h4₄₂₈₋₄₃₁/dES nt 
atgagaccat cagaggctac agtatatctg ccacctgttc ctgtaagcaa agtggtttca
accgatgagt acgtaacacg taccaacatc tactatcacg ctggatctgc gcgcctcctg
actgtcggtc acccatacta ctctattccc aagtcagaca atcccaagaa aatcgtggta
cccaaagtga gcggactcca gtatcgtgtt ttcagagtcc gcttgccaga tcccaacaag
tttggcttcc cagacacaag cttctacaat cctgaaaccc aacgcctggt atgggcatgc
gtgggactcg aggttggccg tggtcagcct ctgggagtgg gcatctcagg ccacccattg
ctcaacaaat tcgatgacac cgagaattcc aacagatacg cgggtggacc aggtacagat
aaccgcgaat gcatcagcat ggactacaag cagacccaac tgtgcctctt gggctgcaag
ccaccaattg gagagcactg gggcaaaggc tcaccttgct ccaacaacgc tatcacacct
ggagactgcc cacccttgga actcaagaat tctgtcattc aggatggtga catggtggac
actggctttg gtgcaatgga tttcaccgct cttcaagaca ccaagtcaaa cgtacctctg
gatatctgca atagcatttg caagtatcca gactacctca agatggttgc tgagccttac
ggtgatacac tgttcttcta cctgagacgt gagcagatgt ttgtgagaca cttcttcaac
cgttccggca ctgtcggaga gtcagttcct aacgacctct acatcaaggg ttctggcagc
acagcaactc tggcgaactc aacctacttt cctactcctt ccggatctat ggtcacgagc
gatgctcaga tcttcaacaa gccctactgg atgcaacgtg cccagggaca caacaatggc
atttgctggg gcaatcagct cttcgtcact gttgtggaca ctactcgctc cactaacatg
tctgtctgcg ctgccattgc caactccgat accactttca aaagctctaa ctttaaggaa
tatctgcgtc acggtgagga gttcgacttg cagttcatct tccaactctg caagatcacc
ctgtccgctg atatcatgac ctacattcac agcatgaatc cagctatcct ggaagactgg
aacttcggtc tgaccactcc accctctggt agcctggagg atacctacag gtttgttaca
tctcaagcaa tcacttgcca gaagctgtac aagacctgca agcaggctgg tacctgccct
cctgacgtga tccctccaca gaagcctaaa gaggacccct tcaaagatta cgtcttctgg
gaggtgaatc tgaaggagaa gttctctgct gatttggatc agtttccact gggtcgtaag
 
ttcctgctcc aagctggata ctaag
SEQ ID No.46
31L1h4₄₂₈₋₄₃₁/dE-CS1 nt 
atgagaccat cagaggctac agtatatctg ccacctgttc ctgtaagcaa agtggtttca
accgatgagt acgtaacacg taccaacatc tactatcacg ctggatctgc gcgcctcctg
actgtcggtc acccatacta ctctattccc aagtcagaca atcccaagaa aatcgtggta
cccaaagtga gcggactcca gtatcgtgtt ttcagagtcc gcttgccaga tcccaacaag
tttggcttcc cagacacaag cttctacaat cctgaaaccc aacgcctggt atgggcatgc
gtgggactcg aggttggccg tggtcagcct ctgggagtgg gcatctcagg ccacccattg
ctcaacaaat tcgatgacac cgagaattcc aacagatacg cgggtggacc aggtacagat
aaccgcgaat gcatcagcat ggactacaag cagacccaac tgtgcctctt gggctgcaag
ccaccaattg gagagcactg gggcaaaggc tcaccttgct ccaacaacgc tatcacacct
ggagactgcc caccettgga actcaagaat tctgtcattc aggatggtga catggtggac
actggctttg gtgcaatgga tttcaccgct cttcaagaca ccaagtcaaa cgtacctctg
gatatctgca atagcatttg caagtatcca gactacctca agatggttgc tgagccttac
ggtgatacac tgttcttcta cctgagacgt gagcagatgt ttgtgagaca cttcttcaac
cgttccggca ctgtcggaga gtcagttcct aacgacctct acatcaaggg ttctggcagc
acagcaactc tggcgaactc aacctacttt cctactcctt ccggatctat ggtcacgagc
gatgctcaga tcttcaacaa gccctactgg atgcaacgtg cccagggaca caacaatggc
atttgctggg gcaatcagct cttcgtcact gttgtggaca ctactcgctc cactaacatg
tctgtctgcg ctgccattgc caactccgat accactttca aaagctctaa ctttaaggaa
tatctgcgtc acggtgagga gttcgacttg cagttcatct tccaactctg caagatcacc
ctgtccgctg atatcatgac ctacattcac agcatgaatc cagctatcct ggaagactgg
aacttcggtc tgaccactcc accctctggt agcctggagg atacctacag gtttgttaca
tctcaagcaa tcacttgcca gaagctgtac aagacctgca agcaggctgg tacctgccct
cctgacgtga tccctaaggt ggagggtcca cagaagccta aagaggaccc cttcaaagat
tacgtcttct gggaggtgaa tctgaaggag aagttctctg ctgatttgga tcagtttcca
ctgggtcgta agttcctgct ccaagctgga taccgtg ctggtccttc gtttgccgct
ggcgcgggtt cggctcctag cgcctcgact accacgccgg ctggcggttc ggccacgggc
agctaag SEQ ID No.47
31L1h4₄₂₈₋₄₃₁/dES-CS1 nt 
atgagaccat cagaggctac agtatatctg ccacctgttc ctgtaagcaa agtggtttca
accgatgagt acgtaacacg taccaacatc tactatcacg ctggatctgc gcgcctcctg
actgtcggtc acccatacta ctctattccc aagtcagaca atcccaagaa aatcgtggta
cccaaagtga gcggactcca gtatcgtgtt ttcagagtcc gcttgccaga tcccaacaag
tttggcttcc cagacacaag cttctacaat cctgaaaccc aacgcctggt atgggcatgc
gtgggactcg aggttggccg tggtcagcct ctgggagtgg gcatctcagg ccacccattg
ctcaacaaat tcgatgacac cgagaattcc aacagatacg cgggtggacc aggtacagat
aaccgcgaat gcatcagcat ggactacaag cagacccaac tgtgcctctt gggctgcaag
ccaccaattg gagagcactg gggcaaaggc tcaccttgct ccaacaacgc tatcacacct
ggagactgcc caccettgga actcaagaat tctgtcattc aggatggtga catggtggac
actggctttg gtgcaatgga tttcaccgct cttcaagaca ccaagtcaaa cgtacctctg
gatatctgca atagcatttg caagtatcca gactacctca agatggttgc tgagccttac
ggtgatacac tgttcttcta cctgagacgt gagcagatgt ttgtgagaca cttcttcaac
cgttccggca ctgtcggaga gtcagttcct aacgacctct acatcaaggg ttctggcagc
 
acagcaactc tggcgaactc aacctacttt cctactcctt ccggatctat ggtcacgagc
gatgctcaga tcttcaacaa gccctactgg atgcaacgtg cccagggaca caacaatggc
atttgctggg gcaatcagct cttcgtcact gttgtggaca ctactcgctc cactaacatg
tctgtctgcg ctgccattgc caactccgat accactttca aaagctctaa ctttaaggaa
tatctgcgtc acggtgagga gttcgacttg cagttcatct tccaactctg caagatcacc
ctgtccgctg atatcatgac ctacattcac agcatgaatc cagctatcct ggaagactgg
aacttcggtc tgaccactcc accctctggt agcctggagg atacctacag gtttgttaca
tctcaagcaa tcacttgcca gaagctgtac aagacctgca agcaggctgg tacctgccct
cctgacgtga tccctccaca gaagcctaaa gaggacccct tcaaagatta cgtcttctgg
gaggtgaatc tgaaggagaa gttctctgct gatttggatc agtttccact gggtcgtaag
ttcctgctcc aagctggata ccgtg ctggtccttc gtttgccgct
ggcgcgggtt cggctcctag cgcctcgact accacgccgg ctggcggttc ggccacgggc
agctaag
SEQ ID No.48
31L1h4₄₂₈₋₄₃₁/dE-CS2 nt 
atgagaccat cagaggctac agtatatctg ccacctgttc ctgtaagcaa agtggtttca
accgatgagt acgtaacacg taccaacatc tactatcacg ctggatctgc gcgcctcctg
actgtcggtc acccatacta ctctattccc aagtcagaca atcccaagaa aatcgtggta
cccaaagtga gcggactcca gtatcgtgtt ttcagagtcc gcttgccaga tcccaacaag
tttggcttcc cagacacaag cttctacaat cctgaaaccc aacgcctggt atgggcatgc
gtgggactcg aggttggccg tggtcagcct ctgggagtgg gcatctcagg ccacccattg
ctcaacaaat tcgatgacac cgagaattcc aacagatacg cgggtggacc aggtacagat
aaccgcgaat gcatcagcat ggactacaag cagacccaac tgtgcctctt gggctgcaag
ccaccaattg gagagcactg gggcaaaggc tcaccttgct ccaacaacgc tatcacacct
ggagactgcc caccettgga actcaagaat tctgtcattc aggatggtga catggtggac
actggctttg gtgcaatgga tttcaccgct cttcaagaca ccaagtcaaa cgtacctctg
gatatctgca atagcatttg caagtatcca gactacctca agatggttgc tgagccttac
ggtgatacac tgttcttcta cctgagacgt gagcagatgt ttgtgagaca cttcttcaac
cgttccggca ctgtcggaga gtcagttcct aacgacctct acatcaaggg ttctggcagc
acagcaactc tggcgaactc aacctacttt cctactcctt ccggatctat ggtcacgagc
gatgctcaga tcttcaacaa gccctactgg atgcaacgtg cccagggaca caacaatggc
atttgctggg gcaatcagct cttcgtcact gttgtggaca ctactcgctc cactaacatg
tctgtctgcg ctgccattgc caactccgat accactttca aaagctctaa ctttaaggaa
tatctgcgtc acggtgagga gttcgacttg cagttcatct tccaactctg caagatcacc
ctgtccgctg atatcatgac ctacattcac agcatgaatc cagctatcct ggaagactgg
aacttcggtc tgaccactcc accctctggt agcctggagg atacctacag gtttgttaca
tctcaagcaa tcacttgcca gaagctgtac aagacctgca agcaggctgg tacctgccct
cctgacgtga tccctaaggt ggagggtcca cagaagccta aagaggaccc cttcaaagat
tacgtcttct gggaggtgaa tctgaaggag aagttctctg ctgatttgga tcagtttcca
ctgggtcgta agttcctgct ccaagctgga tacggcg ctggtccttc gtttgccgct
ggcgcgggtt cggctcctag cgcctcgact accacgccgg ctggcggttc ggccacgggc
agctaag SEQ ID No.49
31L1h4₄₂₈₋₄₃₁/dES-CS2 nt 
atgagaccat cagaggctac agtatatctg ccacctgttc ctgtaagcaa agtggtttca
accgatgagt acgtaacacg taccaacatc tactatcacg ctggatctgc gcgcctcctg
 
actgtcggtc acccatacta ctctattccc aagtcagaca atcccaagaa aatcgtggta
cccaaagtga gcggactcca gtatcgtgtt ttcagagtcc gcttgccaga tcccaacaag
tttggcttcc cagacacaag cttctacaat cctgaaaccc aacgcctggt atgggcatgc
gtgggactcg aggttggccg tggtcagcct ctgggagtgg gcatctcagg ccacccattg
ctcaacaaat tcgatgacac cgagaattcc aacagatacg cgggtggacc aggtacagat
aaccgcgaat gcatcagcat ggactacaag cagacccaac tgtgcctctt gggctgcaag
ccaccaattg gagagcactg gggcaaaggc tcaccttgct ccaacaacgc tatcacacct
ggagactgcc caccettgga actcaagaat tctgtcattc aggatggtga catggtggac
actggctttg gtgcaatgga tttcaccgct cttcaagaca ccaagtcaaa cgtacctctg
gatatctgca atagcatttg caagtatcca gactacctca agatggttgc tgagccttac
ggtgatacac tgttcttcta cctgagacgt gagcagatgt ttgtgagaca cttcttcaac
cgttccggca ctgtcggaga gtcagttcct aacgacctct acatcaaggg ttctggcagc
acagcaactc tggcgaactc aacctacttt cctactcctt ccggatctat ggtcacgagc
gatgctcaga tcttcaacaa gccctactgg atgcaacgtg cccagggaca caacaatggc
atttgctggg gcaatcagct cttcgtcact gttgtggaca ctactcgctc cactaacatg
tctgtctgcg ctgccattgc caactccgat accactttca aaagctctaa ctttaaggaa
tatctgcgtc acggtgagga gttcgacttg cagttcatct tccaactctg caagatcacc
ctgtccgctg atatcatgac ctacattcac agcatgaatc cagctatcct ggaagactgg
aacttcggtc tgaccactcc accctctggt agcctggagg atacctacag gtttgttaca
tctcaagcaa tcacttgcca gaagctgtac aagacctgca agcaggctgg tacctgccct
cctgacgtga tccctccaca gaagcctaaa gaggacccct tcaaagatta cgtcttctgg
gaggtgaatc tgaaggagaa gttctctgct gatttggatc agtttccact gggtcgtaag
ttcctgctcc aagctggata cggcg ctggtccttc gtttgccgct
ggcgcgggtt cggctcctag cgcctcgact accacgccgg ctggcggttc ggccacgggc
agctaag
SEQ ID No.50
31L1h4₄₂₈₋₄₃₁/dE-CS3 nt 
atgagaccat cagaggctac agtatatctg ccacctgttc ctgtaagcaa agtggtttca
accgatgagt acgtaacacg taccaacatc tactatcacg ctggatctgc gcgcctcctg
actgtcggtc acccatacta ctctattccc aagtcagaca atcccaagaa aatcgtggta
cccaaagtga gcggactcca gtatcgtgtt ttcagagtcc gcttgccaga tcccaacaag
tttggcttcc cagacacaag cttctacaat cctgaaaccc aacgcctggt atgggcatgc
gtgggactcg aggttggccg tggtcagcct ctgggagtgg gcatctcagg ccacccattg
ctcaacaaat tcgatgacac cgagaattcc aacagatacg cgggtggacc aggtacagat
aaccgcgaat gcatcagcat ggactacaag cagacccaac tgtgcctctt gggctgcaag
ccaccaattg gagagcactg gggcaaaggc tcaccttgct ccaacaacgc tatcacacct
ggagactgcc cacccttgga actcaagaat tctgtcattc aggatggtga catggtggac
actggctttg gtgcaatgga tttcaccgct cttcaagaca ccaagtcaaa cgtacctctg
gatatctgca atagcatttg caagtatcca gactacctca agatggttgc tgagccttac
ggtgatacac tgttcttcta cctgagacgt gagcagatgt ttgtgagaca cttcttcaac
cgttccggca ctgtcggaga gtcagttcct aacgacctct acatcaaggg ttctggcagc
acagcaactc tggcgaactc aacctacttt cctactcctt ccggatctat ggtcacgagc
gatgctcaga tcttcaacaa gccctactgg atgcaacgtg cccagggaca caacaatggc
atttgctggg gcaatcagct cttcgtcact gttgtggaca ctactcgctc cactaacatg
tctgtctgcg ctgccattgc caactccgat accactttca aaagctctaa ctttaaggaa
tatctgcgtc acggtgagga gttcgacttg cagttcatct tccaactctg caagatcacc
 
ctgtccgctg atatcatgac ctacattcac agcatgaatc cagctatcct ggaagactgg
aacttcggtc tgaccactcc accctctggt agcctggagg atacctacag gtttgttaca
tctcaagcaa tcacttgcca gaagctgtac aagacctgca agcaggctgg tacctgccct
cctgacgtga tccctaaggt ggagggtcca cagaagccta aagaggaccc cttcaaagat
tacgtcttct gggaggtgaa tctgaaggag aagttctctg ctgatttgga tcagtttcca
ctgggtcgta agttcctgct ccaagctgga taccgtg ctggtccttc gtttaaagc
tggcgcgggt tcggctccta gcgcctcgac taccacgccg gctggcggtt cggccacggg
cagctaag SEQ ID No.51
31L1h4₄₂₈₋₄₃₁/dES-CS3 nt

atgagaccat cagaggctac agtatatctg ccacctgttc ctgtaagcaa agtggtttca
accgatgagt acgtaacacg taccaacatc tactatcacg ctggatctgc gcgcctcctg
actgtcggtc acccatacta ctctattccc aagtcagaca atcccaagaa aatcgtggta
cccaaagtga gcggactcca gtatcgtgtt ttcagagtcc gcttgccaga tcccaacaag
tttggcttcc cagacacaag cttctacaat cctgaaaccc aacgcctggt atgggcatgc
gtgggactcg aggttggccg tggtcagcct ctgggagtgg gcatctcagg ccacccattg
ctcaacaaat tcgatgacac cgagaattcc aacagatacg cgggtggacc aggtacagat
aaccgcgaat gcatcagcat ggactacaag cagacccaac tgtgcctctt gggctgcaag
ccaccaattg gagagcactg gggcaaaggc tcaccttgct ccaacaacgc tatcacacct
ggagactgcc caccettgga actcaagaat tctgtcattc aggatggtga catggtggac
actggctttg gtgcaatgga tttcaccgct cttcaagaca ccaagtcaaa cgtacctctg
gatatctgca atagcatttg caagtatcca gactacctca agatggttgc tgagccttac
ggtgatacac tgttcttcta cctgagacgt gagcagatgt ttgtgagaca cttcttcaac
cgttccggca ctgtcggaga gtcagttcct aacgacctct acatcaaggg ttctggcagc
acagcaactc tggcgaactc aacctacttt cctactcctt ccggatctat ggtcacgagc
gatgctcaga tcttcaacaa gccctactgg atgcaacgtg cccagggaca caacaatggc
atttgctggg gcaatcagct cttcgtcact gttgtggaca ctactcgctc cactaacatg
tctgtctgcg ctgccattgc caactccgat accactttca aaagctctaa ctttaaggaa
tatctgcgtc acggtgagga gttcgacttg cagttcatct tccaactctg caagatcacc
ctgtccgctg atatcatgac ctacattcac agcatgaatc cagctatcct ggaagactgg
aacttcggtc tgaccactcc accctctggt agcctggagg atacctacag gtttgttaca
tctcaagcaa tcacttgcca gaagctgtac aagacctgca agcaggctgg tacctgccct
cctgacgtga tccctccaca gaagcctaaa gaggacccct tcaaagatta cgtcttctgg
gaggtgaatc tgaaggagaa gttctctgct gatttggatc agtttccact gggtcgtaag
ttcctgctcc aagctggata ccgtg ctggtccttc gtttaaagc
tggcgcgggt tcggctccta gcgcctcgac taccacgccg gctggcggtt cggccacggg
cagctaag SEQ ID No.52

### Example 5: Construction of recombinant Bacmids and recombinant baculoviruses of genes of L1 proteins and chimeric L1 proteins

The recombinant expression vectors comprising L1 genes, namely pFastBac1-31L1, pFastBac1-T₂₇₄N, pFastBac1-31L1MΔC, pFastBac1-T₂₇₄NΔC, pFastBac1-T₂₆₇AΔC, pFastBac1-T₂₆₇AT₂₇₄NΔC, pFastBac1-T₂₇₄NΔN2C, pFastBac1-T₂₇₄NΔN4C, pFastBac1-T₂₇₄NΔN5C, pFastBac1-T₂₇₄NΔN8C, and pFastBac1-T₂₇₄NΔN10C; or the recombinant expression vectors of chimeric L1 genes, pFastBac1-31L1DE₁₃₂₋₁₃₆/dE, pFastBac1-31L1DE₁₃₂₋₁₃₆/dES, pFastBac1-31L1DE₁₃₂₋₁₃₆/dE-CS1, pFastBac1-31L1DE₁₃₂₋₁₃₆/dES-CS1, pFastBac1-31L1DE₁₃₂₋₁₃₆/dE-CS2, pFastBac1-31L 1DE₁₃₂₋₁₃₆/dES-CS2, pFastBac1-31L1DE₁₃₂₋₁₃₆/dE-CS3, pFastBac1-31L1DE₁₃₂₋₁₃₆/dES-CS3, pFastBac1-31L1h4₄₂₈₋₄₃₁/dE, pFastBac1-31L1h4₄₂₈₋₄₃₁/dES, pFastBac1-31L1h4₄₂₈₋₄₃₁/dE-CS1, pFastBac1-31L1h4₄₂₈₋₄₃₁/dES-CS1, pFastBac1-31L1h4₄₂₈₋₄₃₁/dE-CS2, pFastBac1-31L1h4₄₂₈₋₄₃₁/dES-CS2, pFastBac1-31L1h4₄₂₈₋₄₃₁/dE-CS3, and pFastBac1-31L1h4₄₂₈₋₄₃₁/dES-CS3, were used to transform *E. coli* DH10Bac competent cells, which were screened to obtain recombinant Bacmids. Then the recombinant Bacmids were used to transfect Sf9 insect cells so as to amplify recombinant baculoviruses within the Sf9 cells. Methods of screening of recombinant Bacmids and amplification of recombinant baculoviruses were all well known, for example, the patent CN 101148661 B.

### Example 6: Identification of the expression of genes of L1 proteins and chimeric L1 proteins

Sf9 cells were inoculated with the 11 types of recombinant baculoviruses containing the genes of 31L1 protein or mutants or the 16 types of recombinant baculoviruses containing the chimeric L1 genes, respectively, to express the proteins. After incubation at 27°C for about 88 h, the fermentation broth was collected and centrifuged at 3,000 rpm for 15 min. The supernatant was discarded, and the cells were washed with PBS for use in expression identification and purification. Methods of infection and expression were publicly available, for example, the patent CN 101148661 B.

### Example 7: Identification of the expression of L1 proteins and chimeric L1 proteins

For each of cells expressing the different L1 proteins or chimeric L1 proteins described in Example 6, 1×10⁶ cells were collected and resuspended in 200 µl PBS solution. 50 µl of 6× Loading Buffer was added and the samples were denatured at 75°C for 8 minutes. 10 µl of sample was used for SDS-PAGE electrophoresis and Western blot identification, respectively. The results were as shown in Figures 1A to 1B. The 11 types of 31L1 protein or mutants and 16 types of chimeric L1 proteins could all be expressed at high levels in insect cells, among which the protein size of 31L1, T₂₇₄N, 31L1DE₁₃₂₋₁₃₆/dE, 31L1DE₁₃₂₋₁₃₆/dES, 31L1h4₄₂₈₋₄₃₁/dE, and 31L1h4₄₂₈₋₄₃₁/dES was about 55 kDa, the protein size of 31L1MΔC, T₂₇₄NΔC, T₂₆₇AΔC, T₂₆₇AT₂₇₄NΔC, T₂₇₄NΔN2C, T₂₇₄NΔN4C, T₂₇₄NΔN5C, T₂₇₄NΔN8C, and T₂₇₄NΔN10C was about 50 kD, the protein size of 31L1DE₁₃₂₋₁₃₆/dE-CS1, 31L1DE₁₃₂₋₁₃₆/dES-CS1, 31L1DE₁₃₂₋₁₃₆/dE-CS2, 31L1DE₁₃₂₋₁₃₆/dES-CS2, 31L1DE₁₃₂₋₁₃₆/dES-CS3, 31L1DE₁₃₂₋₁₃₆/dES-CS3, 31L1h4₄₂₈₋₄₃₁/dE-CS1, 31L1h4₄₂₈₋₄₃₁/dES-CS1, 31L1h4₄₂₈₋₄₃₁/dE-CS2, 31L1h4₄₂₈₋₄₃₁/dES-CS2, 31L1h4₄₂₈₋₄₃₁/dE-CS3, and 31L1h4₄₂₈₋₄₃₁/dES-CS3a was about 59 kDa. Methods of SDS-PAGE electrophoresis and Western blot identification were publicly available, for example, the patent CN 101148661 B.

### Example 8: Comparison of the expression amounts of L1 proteins and chimeric L1 proteins in insect cells

For each of cells expressing the different recombinant proteins described in Example 6, 1×10⁶ cells were collected and resuspended in 200 µl PBS solution. The cells were disrupted by ultrasonic disruption (Ningbo Scientz Ultrasonic Cell Disruptor, 2# probe, 100 W, ultrasound 5 s, interval 7 s, total time 3 min) and centrifuged at a high speed of 12,000 rpm for 10 minutes. The lysed supernatant was collected and the L1 content in the supernatant was detected by sandwich ELISA, which was well known, for example, the patent CN104513826A.

Microtiter plates were coated with HPV31L1 monoclonal antibodies prepared by the inventor at 80 ng/well by overnight incubation at 4°C. The plate was blocked with 5% BSA-PBST at room temperature for 2 h and washed for 3 times with PBST. The lysed supernatant was subjected to 2-fold serial dilution with PBS. The HPV31L1 VLP standard was also subjected to serial dilution from a concentration of 2 µg/ml to 0.0625 µg/ml. The diluted samples were added to the plate respectively at 100 µl per well and incubated at 37°C for 1 h. The plate was washed for 3 times with PBST, and 1:3000 diluted HPV31L1 rabbit polyclonal antibody was added at 100 µl per well and incubated at 37°C for 1 h. The plate was washed for 3 times with PBST, and 1:3000 diluted HRP-labeled goat anti-mouse IgG (1:3000 dilution, ZSGB-Bio Corporation) was added and incubated at 37°C for 45 minutes. The plate was washed for 5 times with PBST, and 100 µl of OPD substrate (Sigma) was added to each well for chromogenic reaction at 37°C for 5 minutes. The reaction was stopped with 50 µl of 2 M sulfuric acid, and the absorbance at 490 nm was determined. The concentrations of the 31L1 protein, mutants of the 31L1 protein or chimeric L1 proteins in the lysed supernatant were calculated according to the standard curve.

As shown in Table 4, the expression amount of the 31L1 mutant protein with a 29-amino acid truncation at the C-terminus of the present invention (31L1MΔC) was significantly higher than that of the HPV31L1 full-length protein. The expression amounts of the mutant proteins obtained by point mutation of the 31L1 protein also varied, among which the expression amount of the T₂₇₄N mutant was significantly higher than that of the original HPV31L1 protein, and the expression amount of the T₂₇₄NΔC mutant protein was further increased than that of the 31L1MΔC protein, indicating that the mutation of threonine at position 274 to asparagine could increase the expression amount of the 31L1 protein. Different N-terminus truncations were performed on the basis of T₂₇₄NΔC, and it was found that different truncations had different effects on the expression amount, among which the expression amounts of truncation mutations obtained by a 4-amino acid truncation at the N-terminus (T₂₇₄NΔN4C) or an 8-amino acid truncation at the N-terminus (T₂₇₄NΔN8C) were 2 folds and 1.28 folds that of T₂₇₄NΔC, respectively. The expression amounts of the chimeric proteins (31L1DE₁₃₂₋₁₃₆/dE, 31L1DE₁₃₂₋₁₃₆/dES, 31L1h4₄₂₈₋₄₃₁/dE, 31L1h4₄₂₈₋₄₃₁/dES) constructed on the basis of T₂₇₄NΔN4C were all comparable to that of their backbone T₂₇₄NΔN4C. In addition, the expression amounts of 12 types of chimeric proteins with the 31L1 mutant with C-terminus substitutions as the backbone were all higher than that of the corresponding chimeric protein with C-terminus truncation.

**Table 4. Analysis of the expression amounts of 31L1 protein, 31L1 protein mutants and chimeric L1 proteins**

| Protein name | Expression amount (mg/L) | | | |
|---|---|---|---|---|
| | Batch 1 | Batch 2 | Batch 3 | Average |
| HPV31L1 | 19 | 25 | 15 | 19.7 |
| T₂₇₄N | 38 | 40 | 36 | 38 |
| 31L1MΔC | 35 | 38 | 30 | 34.3 |
| T₂₇₄NΔC | 59 | 59 | 52 | 56.7 |
| T₂₆₇AΔC | 22 | 28 | 27 | 25.7 |
| T₂₆₇AT₂₇₄NΔC | 14 | 12 | 18 | 14.6 |
| T₂₇₄NΔN2C | 29 | 27 | 26 | 27.3 |
| T₂₇₄NΔN4C | 115 | 108 | 120 | 114.3 |
| T₂₇₄NΔN5C | 30 | 32 | 33 | 31.7 |
| T₂₇₄NΔN8C | 70 | 75 | 73 | 72.7 |
| T₂₇₄NΔN10C | 11 | 15 | 12 | 12.7 |
| 31L1DE₁₃₂₋₁₃₆/dE | 102 | 125 | 119 | 115.3 |
| 31L1DE₁₃₂₋₁₃₆/dES | 128 | 133 | 122 | 127.6 |
| 31L1DE₁₃₂₋₁₃₆/dE-CS1 | 154 | 152 | 160 | 155.3 |
| 31L1DE₁₃₂₋₁₃₆/dES-CS1 | 173 | 141 | 139 | 151 |
| 31L1DE₁₃₂₋₁₃₆/dE-CS2 | 158 | 162 | 155 | 158.3 |
| 31L1DE₁₃₂₋₁₃₆/dES-CS2 | 157 | 143 | 140 | 146.7 |
| 31L1DE₁₃₂₋₁₃₆/dE-CS3 | 163 | 182 | 145 | 163.3 |
| 31L1DE₁₃₂₋₁₃₆/dES-CS3 | 171 | 157 | 166 | 164.7 |
| 31L1h4₄₂₈₋₄₃₁/dE | 112 | 118 | 117 | 115.7 |
| 31L1h4₄₂₈₋₄₃₁/dES | 115 | 123 | 125 | 121 |
| 31L1h4₄₂₈₋₄₃₁/dE-CS1 | 182 | 130 | 155 | 155.7 |
| 31L1h4₄₂₈₋₄₃₁/dES-CS1 | 173 | 148 | 170 | 163.7 |
| 31L1h4₄₂₈₋₄₃₁/dES-CS2 | 149 | 162 | 151 | 154 |
| 31L1h4₄₂₈₋₄₃₁/dE-CS2 | 154 | 158 | 150 | 154 |
| 31L1h4₄₂₈₋₄₃₁/dE-CS3 | 162 | 143 | 148 | 151 |
| 31L1h4₄₂₈₋₄₃₁/dES-CS3 | 151 | 160 | 154 | 155 |

### Example 9: Purification and dynamic light scattering particle size analysis of L1 proteins and chimeric L1 proteins

An appropriate amount of cell fermentation broth of the above recombinant proteins was collected and the cells were resuspended with 10 ml PBS. PMSF was added to a final concentration of 1 mg/ml. The cells were ultrasonically disrupted (Ningbo Scientz Ultrasonic Cell Disruptor, 6# probe, 200 W, ultrasound 5 s, interval 7 s, total time 10 min) and the disrupted supernatant was collected for purification. The purification steps were carried out at room temperature. 4% β-mercaptoethanol (w/w) was added to the lysate to disaggregate VLP. Then the samples were filtered with 0.22 µm filters, followed by successive purification with DMAE anion exchange chromatography or CM cation exchange chromatography (20 mM Tris, 180 mM NaCl, 4% β-ME, elution at pH 7.9), TMAE anion exchange chromatography or Q cation exchange chromatography (20 mM Tris, 180 mM NaCl, 4% β-ME, elution at pH 7.9) and hydroxyapatite chromatography (100 mM NaH₂PO₄, 30 mM NaCl, 4% β-ME, elution at pH 6.0). The purified product was concentrated and buffer (20 mM NaH₂PO₄, 500 mM NaCl, pH 6.0) exchange was performed using Planova ultrafiltration system to prompt VLP assembly. The above purification methods were all publicly available, for example the patents CN101293918B, CN1976718A, etc.

The purified HPV31L1 protein, 31L1 mutant proteins and chimeric L1 proteins could all be effectively assembled. The solutions of the assembled proteins were subjected to DLS particle size analysis (Zetasizer Nano ZS 90 Dynamic Light Scattering Analyzer, Malvern), and the results were as shown in Table 5. Among them, the DLS analysis plots of 31L1MΔC, T₂₇₄NΔC, T₂₇₄NΔN4C, 31L1DE₁₃₂₋₁₃₆/dE, and 31L1h4₄₂₈₋₄₃₁/dE were as shown in Figures 2A to 2F.

**Table 5. DLS analysis of L1 proteins and chimeric L1 proteins**

| Protein name | Hydraulic diameter (nm) | PDI |
|---|---|---|
| HPV31L1 | 102.5 | 0.128 |
| T₂₇₄N | 104.2 | 0.192 |
| 31L1MΔC | 103.3 | 0.190 |
| T₂₇₄NΔC | 99.78 | 0.169 |
| T₂₆₇AΔC | 101.4 | 0.187 |
| T₂₆₇AT₂₇₄NΔC | 98.2 | 0.188 |
| T₂₇₄NΔN2C | 105.8 | 0.166 |
| T₂₇₄NΔN4C | 106.8 | 0.172 |
| T₂₇₄NΔN5C | 102.4 | 0.127 |
| T₂₇₄NΔN8C | 100.9 | 0.153 |
| T₂₇₄NΔN10C | 97.55 | 0.132 |
| 31L1DE₁₃₂₋₁₃₆/dE | 104.59 | 0.192 |
| 31L1DE₁₃₂₋₁₃₆/dES | 108.5 | 0.183 |
| 31L1DE₁₃₂₋₁₃₆/dE-CS1 | 109.4 | 0.112 |
| 31L1DE₁₃₂₋₁₃₆/dES-CS1 | 108.8 | 0.146 |
| 31L1DE₁₃₂₋₁₃₆/dE-CS2 | 103.2 | 0.159 |
| 31L1DE₁₃₂₋₁₃₆/dES-CS2 | 105.7 | 0.182 |
| 31L1DE₁₃₂₋₁₃₆/dE-CS3 | 117.4 | 0.193 |
| 31L1DE₁₃₂₋₁₃₆/dES-CS3 | 116.2 | 0.162 |
| 31L1h4₄₂₈₋₄₃₁/dE | 47.8 | 0.267 |
| 31L1h4₄₂₈₋₄₃₁/dES | 39.6 | 0.201 |
| 31L1h4₄₂₈₋₄₃₁/dE-CS1 | 42.4 | 0.196 |
| 31L1h4₄₂₈₋₄₃₁/dES-CS1 | 36.7 | 0.175 |
| 31L1h4₄₂₈₋₄₃₁/dES-CS2 | 45.3 | 0.173 |
| 31L1h4₄₂₈₋₄₃₁/dE-CS2 | 49.2 | 0.211 |
| 31L1h4₄₂₈₋₄₃₁/dE-CS3 | 46.1 | 0.156 |
| 31L1h4₄₂₈₋₄₃₁/dES-CS3 | 38.4 | 0.133 |

### Example 10: Transmission electron microscopy observation of VLPs and chimeric VLPs

The recombinant proteins were purified separately according to the chromatographic purification method described in Example 9. The assembled chimeras were prepared on copper mesh, stained with 1% uranium acetate, fully dried and then observed using JEM-1400 electron microscope (Olympus). The results showed that the HPV31L1, T₂₇₄N, 31L1MΔC, T₂₇₄NΔC, T₂₆₇AΔC and T₂₆₇AT₂₇₄NΔC proteins expressed by insect cells could all be assembled into VLPs with a diameter of about 50-60 nm. The mutants of the 31L1 protein with N-terminal truncation in combination with C-terminal truncation could be assembled into VLPs with a diameter of 17-35 nm. The chimeric proteins with insertion of 73L2 polypeptide in the surface region of the DE loop could be assembled into cVLPs of 30-50 nm. The chimeric proteins with insertion of 73L2 polypeptide in the h4 region could be assembled into cVLPs with a diameter of approximately 17-30 nm. The electron microscopy images of VLPs or cVLPs of 31L1MΔC, T₂₇₄NΔN4C, 31L1DE₁₃₂₋₁₃₆/dE, 31L1h4₄₂₈₋₄₃₁/dE, and 31L1h4₄₂₈₋₄₃₁/dE-CS1 were as shown in Figures 3A to 3E. Methods of copper mesh preparation and electron microscopy observation were all publicly available, for example, the patent CN 101148661 B.

### Example 11: Immunization of mice with HPV31L1 or mutant VLPs and determination of neutralizing antibody titers

4-6 weeks old BALB/c mice were randomly divided into groups, 5 mice per group, and immunized with 0.1µg VLP. VLP was subcutaneously injected at Week 0 and Week 2 for a total of 2 doses. Tail vein blood was collected 2 weeks after the second immunization and serum was isolated.

The neutralizing antibody titers of immune serum were detected using HPV31 pseudovirus, and the VLP-immunized mice of various 31L1 mutants showed that the levels of HPV31-specific neutralizing antibodies were comparable to those of the prototype. The immunization results of 31L1MΔC, T274NΔC, and T274NΔN4C are shown in Figure 4.

### Example 12: Immunization of mice with chimeric VLPs and determination of neutralizing antibody titers

4-6 weeks old BALB/c mice were randomly divided into groups, 5 mice in each group, and 10 µg cVLP in combination with 50 µg Al(OH)₃ and 5 µg MPL adjuvant were used to immunize the mice by subcutaneous injection at Weeks 0, 4, 7, and 10, for a total of 4 times. Tail vein blood was collected 2 weeks after the 4th immunization and serum was isolated.

17 types of HPV pseudoviruses were used to detect the neutralizing antibody titers of the antiserum. The results showed that after immunizing mice with various cVLPs, the levels and neutralization range of the induced cross-neutralizing antibodies were different with each other. Among them, the neutralizing antibody titers of the backbone type HPV type 31 induced by cVLPs with chimeric epitopes in the surface region of h4 were comparable to that of HPV31L1VLP, and their antiserum had high titers of cross-neutralizing antibodies, which could neutralize the 17 types of pseudoviruses used for detection. The neutralizing antibody titers of HPV type 31 induced by cVLPs with chimeric epitopes in the surface region of DE loop were reduced by 1 order of magnitude compared with that of 31L1VLP, and the cross-neutralization spectrum of their immune serum was relatively narrow. The cross-neutralization activities of some cVLP immune serum were as shown in Table 5, in which 31L1h4₄₂₈₋₄₃₁/dE, 31L1h4₄₂₈₋₄₃₁/dES and 31L1h4₄₂₈₋₄₃₁/dE-CS1 antiserum could neutralize at least 17 types of pseudoviruses, and 31L1DE₁₃₂₋₁₃₆/dE and 31L1DE₁₃₂₋₁₃₆/dES antiserum only neutralized 10 and 8 types of pseudoviruses, respectively. It was worth mentioning that the neutralizing titers of 31L1h4₄₂₈₋₄₃₁/dE, 31L1h4₄₂₈₋₄₃₁/dES and 31L1h4₄₂₈₋₄₃₁/dE-CS1 antiserum against HPV types 16, -18 and -45 were all greater than 10³, and the neutralizing antibody titer of 31L1h4₄₂₈₋₄₃₁/dE-CS1 antiserum against HPV type 73 was also greater than 10³.

In addition, in the present invention, after immunizing mice with the above strategy using the cVLPs constructed with the 31L1 mutant with C-terminus substitutions, the levels and neutralization ranges of the induced cross-neutralizing antibodies were consistent with the corresponding C-terminus truncated cVLPs.

Methods of pseudovirus preparation and pseudoviral neutralization experiments were all publicly available, for example, the patent CN 104418942A.

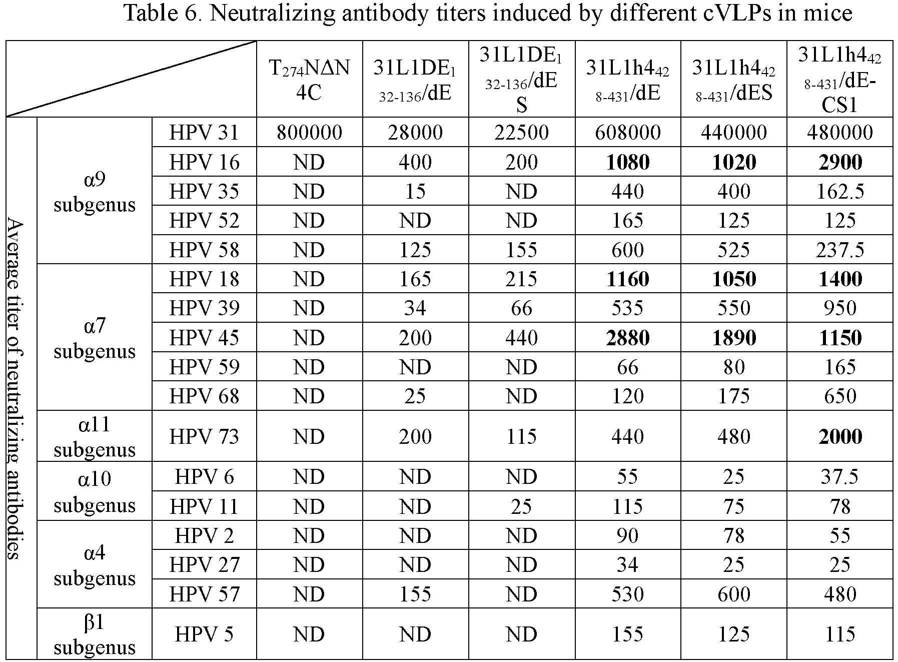

## Claims

1. A human papillomavirus chimeric protein comprising or consisting of a HPV type 31 L1 protein or a mutant of the HPV type 31 L1 protein, and a polypeptide from a HPV type 73 L2 protein inserted into the HPV type 31 L1 protein or the mutant of the HPV type 31 L1 protein, wherein the HPV type 31 L1 protein is as shown in SEQ ID No. 1, and the HPV type 73 L2 protein is as shown in SEQ ID No. 2;
preferably, the polypeptide from the HPV type 73 L2 protein is as shown in SEQ ID No. 15, SEQ ID No. 16 or SEQ ID No.17;
further preferably, the polypeptide from the HPV type 73 L2 protein is inserted into the surface region of the HPV type 31 L1 protein or the mutant of the HPV type 31 L1 protein, preferably inserted into the DE loop or h4 region of the HPV type 31 L1 protein or the mutant of the HPV type 31 L1 protein;
more preferably, the polypeptide from the HPV type 73 L2 protein is inserted between amino acids 132 and 133, or between amino acids 134 and 135, or between amino acids 136 and 137, or between amino acids 137 and 138, or between amino acids 432 and 433, or between amino acids 434 and 435, or between amino acids 435 and 436 of the HPV type 31 L1 protein or the mutant of the HPV type 31 L1 protein by direct insertion; alternatively inserted into the region of amino acids 132 to 136, or the region of amino acids 135 to 139, or the region of amino acids 428 to 431, or the region of amino acids 431 to 434 of the HPV type 31 L1 protein or the mutant of the HPV type 31 L1 protein by non-isometric substitution;
more preferably, the polypeptide from the HPV type 73 L2 protein comprises a linker of 1 to 3 amino acid residues in length at its N-terminus and/or C-terminus, more preferably, the linker comprises 1 to 3 amino acids selected from the group consisting of glycine, serine, alanine and proline; more preferably, the linker at the N-terminus consists of glycine-proline, and the linker at the C-terminus consists of proline;
further preferably, the mutant of the HPV type 31 L1 protein comprises any one or more mutations selected from i) to iii), compared with the HPV type 31 L1 protein as shown in SEQ ID No. 1:
i) any one or more substitution mutation(s) selected from the group consisting of T274N, R475G, R483G, R496G, K477S, K497S, K501S, K479A, K482A, K498A, K495G, K500G and R473G;
ii) truncation mutation of 2, 4, 5, 8 or 10 amino acids truncated at the N-terminus; and
iii) truncation mutation of 29 amino acids truncated at the C-terminus;
further preferably, the mutant of the HPV type 31 L1 protein is any one selected from the variants as shown in SEQ ID Nos. 3, 4, 5, 6, 7, 8, 9, 10, 11, 12, 13 and 14.

2. The human papillomavirus chimeric protein according to claim 1, wherein the amino acid sequence of the human papillomavirus chimeric protein is as shown in any one of SEQ ID Nos. 18-33.

3. A polynucleotide encoding the human papillomavirus chimeric protein according to claim 1 or 2, preferably, the sequence of the polynucleotide is whole-gene optimized using *E. coli* codons or whole-gene optimized using insect cell codons, more preferably, the sequence of the polynucleotide is as shown in any one of SEQ ID No. 37 to SEQ ID No. 52.

4. A vector comprising the polynucleotide according to claim 3.

5. A cell comprising the vector according to claim 4.

6. A polymer which is a chimeric pentamer or chimeric virus-like particle comprising the human papillomavirus chimeric protein according to claim 1 or 2, or formed by the human papillomavirus chimeric protein according to claim 1 or 2.

7. Use of the human papillomavirus chimeric protein according to claim 1 or 2 or the polymer according to claim 6 in the preparation of a vaccine for the prevention of papillomavirus infection and/or papillomavirus infection-induced diseases, preferably, the papillomavirus infection-induced diseases are selected from the group consisting of cervical cancer, vaginal cancer, vulval cancer, penile cancer, perianal cancer, oropharyngeal cancer, tonsil cancer and oral cancer, preferably, the subject is a human subj ect;
preferably, the papillomavirus infection is an infection selected from one or more of the following papillomavirus types: HPV16, HPV18, HPV26, HPV31, HPV33, HPV35, HPV39, HPV45, HPV51, HPV52, HPV53, HPV56, HPV58, HPV59, HPV66, HPV68, HPV70, HPV73, HPV6, HPV11, HPV2, HPV5, HPV27 and HPV57.

8. A vaccine for the prevention of papillomavirus infection and/or a papillomavirus infection-induced disease, comprising the human papillomavirus chimeric protein according to claim 1 or 2 or the polymer according to claim 6, an adjuvant, as well as an excipient or carrier for vaccines.

9. The vaccine for the prevention of papillomavirus infection and/or a papillomavirus infection-induced disease according to claim 8, further comprising at least one virus-like particle or chimeric virus-like particle of HPV of the mucosa-tropic group and/or skin-tropic group.

10. The vaccine for the prevention of papillomavirus infection and/or a papillomavirus infection-induced disease according to claim 8 or 9, wherein the adjuvant is an adjuvant for human use.
